# EUROPÄISCHE PATENTSCHRIFT

(11) **EP 1 301 520 B1**
(45) Veröffentlichungstag und Bekanntmachung des Hinweises auf die Patenterteilung: **20.09.2006**
(21) Anmeldenummer: 01965129.8
(22) Anmeldetag: 13.07.2001
(51) Int. Cl.: C07F 15/04, C07D 207/50, C08F 4/70, C08F 10/00

(54) **LIGANDEN, KOMPLEXVERBINDUNGEN UND IHRE VERWENDUNG ZUR POLYMERISATION VON OLEFINEN**
LIGANDS, COMPLEX COMPOUNDS AND THEIR USE FOR POLYMERISING OLEFINS
LIGANDS, COMPOSES COMPLEXES ET LEUR UTILISATION POUR LA POLYMERISATION D'OLEFINES

(30) Priorität: 20.07.2000 DE 10035654
(43) Veröffentlichungstag der Anmeldung: 16.04.2003
(73) Patentinhaber: BASF Aktiengesellschaft, 67056 Ludwigshafen (DE)
(72) Erfinder: KRISTEN, Marc, Oliver, 67117 Limburgerhof (DE); BILDSTEIN, Benno, A-6020 Innsbruck (AT); AMORT, Christoph, I-39030 Rodeneck (IT); MALAUN, Michael, 80469 München (DE)
(74) Vertreter: Bieller, Vera
(86) Internationale Anmeldenummer: PCT/EP2001/008113
(87) Internationale Veröffentlichungsnummer: WO 2002/008236

(56) Entgegenhaltungen:
- WO-A-00/50470
- WO-A-98/30609
- WO-A-98/42664
- DATABASE CA [Online] CHEMICAL ABSTRACTS SERVICE, COLUMBUS, OHIO, US; COLAUTTI, A. ET AL: "New coccidiostatic drugs of the 4-amino-4H-1,2,4-triazole series" retrieved from STN Database accession no. 76:99572 XP002181151 & CHIM. THER. (1971), 6(5), 367-79 ,
- DATABASE CA [Online] CHEMICAL ABSTRACTS SERVICE, COLUMBUS, OHIO, US; KUZNETSOV, L. I. ET AL: "Azomethines of N-aminobenzazoles as a novel ligand system" retrieved from STN Database accession no. 97:16100 XP002181152 & KOORD. KHIM. (1982), 8(4), 445-53 ,
- DATABASE CA [Online] CHEMICAL ABSTRACTS SERVICE, COLUMBUS, OHIO, US; GILYANOVSKII, P. V. ET AL: "Luminescence and photochemistry of metal complexes with 4-salicylideneamino-1,2,4-triazole" retrieved from STN Database accession no. 103:133931 XP002181153 & KOORD. KHIM. (1985), 11(7), 889-95 ,
- DATABASE CA [Online] CHEMICAL ABSTRACTS SERVICE, COLUMBUS, OHIO, US; BACH, A. ET AL: "Metal chelates of N-(1-pyrrolyl)salicylaldimines and their structure determination by x-ray structure analysis and x-ray absorption spectroscopy (XANES)" retrieved from STN Database accession no. 125:211201 XP002181154 & Z. NATURFORSCH., B: CHEM. SCI. (1996), 51(6), 757-764 ,
- DATABASE CA [Online] CHEMICAL ABSTRACTS SERVICE, COLUMBUS, OHIO, US; WANG, CHANGFENG ET AL: "Synthesis and properties of copper complexes of symmetrical triazole Schiff bases" retrieved from STN Database accession no. 127:184821 XP002181155 & YINGYONG HUAXUE (1997), 14(3), 29-32 ,

## Beschreibung

Die vorliegende Erfindung betrifft Komplexverbindungen der allgemeinen Formel I, 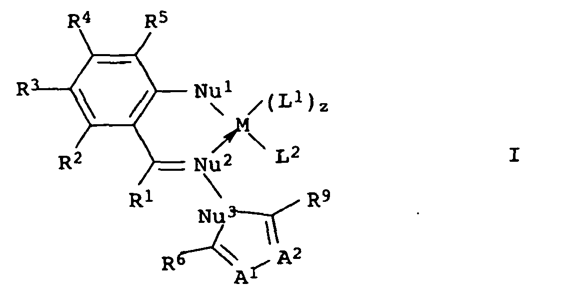 bei denen die Variablen wir folgt definiert sind:
- M: ein Element der 6. bis 10. Gruppe des Periodensystems der Elemente, ausser Nickel
- Nu¹: ausgewählt aus O, S oder Se;
- Nu², Nu³: ausgewählt aus N oder P,
- A¹: N oder C-R⁷ oder Si-R⁷,
- A²: N oder C-R⁸ oder Si-R⁸,
- R¹ bis R⁹: ausgewählt aus
Wasserstoff,
C₁-C₈-Alkyl, substituiert oder unsubstituiert,
C₂-C₈-Alkenyl, substituiert oder unsubstituiert, mit ein bis 4 isolierten oder konjugierten Doppelbindungen;
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy, substituiert oder unsubstituiert,
C₆-C₁₄-Aryloxy,
SiR¹⁰R¹¹R¹² oder O-SiR¹⁰R¹¹R¹²;
fünf- bis sechsgliedrigen stickstoffhaltigen Heteroarylresten, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy,
C₆-C₁₄-Aryloxy,
SiR¹⁰R¹¹R¹² oder O-SiR¹⁰R¹¹R¹²;
wobei räumlich benachbarte Reste R¹ bis R⁹ miteinander zu einem 5-bis 12-gliedrigen Ring verbunden sein können;
- L¹: ein organischer oder anorganischer Neutralligand,
- L²: ein organischer oder anorganischer anionischer Ligand, wobei L¹ und L² miteinander durch eine oder mehrere kovalente Bindungen verknüpft sein können,
- z: eine ganze Zahl von 1 bis 3,
- R¹⁰ bis R¹²: gleich oder verschieden und ausgewählt werden aus Wasserstoff, C₁-C₈-Alkyl, C₃-C₁₂-Cycloalkyl, C₇-C₁₃-Aralkyl oder C₆-C₁₄-Aryl.

Weiterhin betrifft die vorliegende Erfindung Komplexverbindungen der allgemeinen Formel I 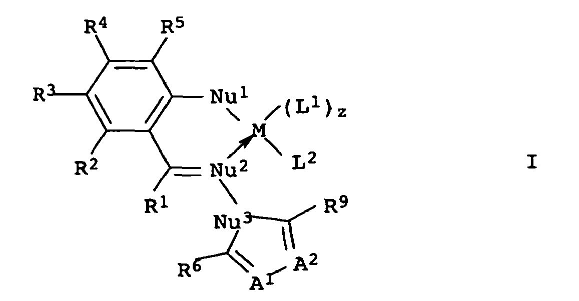 bei denen die Variablen wir folgt definiert sind:
- M: Nickel,
- Nu¹: ausgewählt aus O, S oder Se;
- Nu², Nu³: ausgewählt aus N oder P,
- A¹: N oder Si-R⁷,
- A²: N oder C-R⁸ oder Si-R⁸,
- R¹ bis R⁹: ausgewählt aus Wasserstoff,
C₁-C₈-Alkyl, substituiert oder unsubstituiert,
C₂-C₈-Alkenyl, substituiert oder unsubstituiert, mit ein bis 4 isolierten oder konjugierten Doppelbindungen;
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy, substituiert oder unsubstituiert,
C₆-C₁₄-Aryloxy,
SiR¹⁰R¹¹R¹² oder O-SiR¹⁰R¹¹R¹²;
fünf- bis sechsgliedrigen stickstoffhaltigen Heteroarylresten, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy,
C₆-C₁₄-Aryloxy,
SiR¹⁰R¹¹R¹² oder O-SiR¹⁰R¹¹R¹²;
wobei räumlich benachbarte Reste R¹ bis R⁹ miteinander zu einem 5-bis 12-gliedrigen Ring verbunden sein können;
- L¹: ein organischer oder anorganischer Neutralligand,
- L²: ein organischer oder anorganischer anionischer Ligand, wobei L¹ und L² miteinander durch eine oder mehrere kovalente Bindungen verknüpft sein können,
- z: eine ganze Zahl von 1 bis 3,
- R¹⁰ bis R¹²: gleich oder verschieden und ausgewählt werden aus Wasserstoff, C₁-C₈-Alkyl, C₃-C₁₂-Cycloalkyl, C₇-C₁₃-Aralkyl oder C₆-C₁₄-Aryl.

Weiterhin betrifft die vorliegende Erfindung ein Verfahren zur Herstellung der erfindungsgemäßen Komplexverbindungen aus Liganden der allgemeinen Formel II

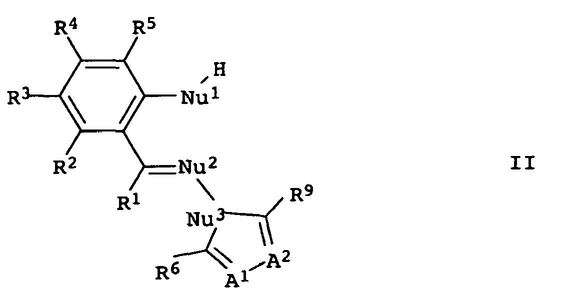

Weiterhin betrifft die vorliegende Erfindung ein Verfahren zur Herstellung von Trägerkatalysatoren für die Polymerisation unter Verwendung der erfindungsgemäßen Komplexverbindung der allgemeinen Formeln I sowie ein Verfahren zur Polymerisation oder Copolymerisation von Olefinen unter Verwendung der erfindungsgemäßen Trägerkatalysatoren.

Schließlich betrifft die vorliegende Erfindung ein Verfahren zur Emulsionspolymerisation und -Copolymerisation von Olefinen unter Verwendung einer Komplexverbindung der allgemeinen Formel I, worin M ein Element der 6. bis 10. Gruppe des Periodensystems der Elemente ist.

Polymere und Copolymere von Olefinen sind wirtschaftlich von großer Bedeutung, weil die Monomere in großen Mengen leicht zugänglich sind und weil sich die Polymere durch Variation des Herstellverfahrens oder der Verarbeitungsparameter in weiten Bereichen variieren lassen. Besondere Aufmerksamkeit beim Herstellverfahren gilt dabei dem verwendeten Katalysator. Neben Ziegler-Natta-Katalysatoren sind verschiedenartige Single-Site-Katalysatoren von wachsender Bedeutung, wobei als Zentralatome neben Zr wie beispielsweise in Metallocenkatalysatoren (H.-H. Brintzinger et al., Angew. Chem. 1995, 107, 1255) auch Ni oder Pd (WO 96/23010) oder Fe und Co (z.B. WO 98/27124) genauer untersucht worden sind. Die Komplexe von Ni, Pd, Fe und Co werden auch als Komplexe später Übergangsmetalle bezeichnet.

Metallocenkatalysatoren haben für den großtechnischen Einsatz Nachteile. Die am häufigsten verwendeten Metallocene, das sind Zirkonocene und Hafnocene, sind hydrolyseempfindlich. Außerdem sind die meisten Metallocene empfindlich gegenüber einer Vielzahl von Katalysatorgiften wie beispielsweise Alkoholen, Ethern oder CO, was eine sorgfältige Reinigung der Monomeren bedingt.

Während Ni- oder Pd-Komplexe (WO 96/23010) die Bildung hochverzweigter, kommerziell wenig interessanter Polymere katalysieren, führt die Verwendung von Fe- oder Co-Komplexen zur Bildung von hochlinearem Polyethylen mit sehr geringen Anteilen an Comonomer.

Weiterhin sind Komplexe untersucht worden, mit Hilfe derer sich Ethylen in Anwesenheit von Wasser polymerisieren oder copolymerisieren lässt.

In WO 98/42664 sind Komplexe der Formel A 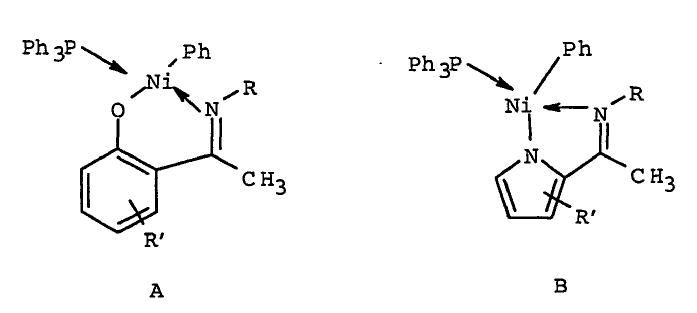 und eng verwandte Derivate mit Salicylaldiminliganden beschrieben sowie ihre Verwendung zur Polymerisation von Olefinen.

In WO 98/42665 sind Komplexe der Formel B und eng verwandte Derivate beschrieben sowie ihre Verwendung zur Polymerisation von Olefinen.

Gemeinsam ist den Komplexen der Formeln A und B, dass der Rest R am Imin-Stickstoff eine C₁-C₁₁-Alkylgruppe oder eine ortho-substituierte Phenylgruppe bedeutet. Ihre Aktivität sollte sich jedoch noch verbessern lassen.

Von den Komplexen der allgemeinen Formel A und B ist weiterhin bekannt, dass sie auch in Anwesenheit geringer Mengen von Wasser polymerisationsaktiv sind, ohne dass die katalytische Aktivität Einbußen erleidet (WO 98/42664, insbesondere Seite 17, Zeile 14 ff; WO 98/42665, S. 16, Zeile 13). Diese Wassermengen dürfen jedoch 100 Äquivalente, bezogen auf den Komplex, nicht überschreiten (WO 98/42664, Seite 17, Zeile 33-35; WO 98/42665, Seite 16, Zeile 30-31). Unter diesen Bedingungen kann jedoch keine Emulsionspolymerisation durchgeführt werden.

In WO 98/30609 sind Derivate von A offengelegt, die sich zur Polymerisation von Ethylen und Propylen eignen. Ihre Aktivität ist indes nicht immer befriedigend.

In EP-A 0 874 005 werden weitere polymerisationsaktive Komplexe offengelegt. Es handelt sich bei den Komplexen bevorzugt um Ti-Komplexe mit Salicylaldiminliganden. Auch sie tragen Phenylsubstituenten oder substituierte Phenylsubstituenten am Aldimin-Stickstoffatom (Seite 18-23) oder aber das Aldimin-Stickstoffatom ist in einen 6-gliedrigen Ring eingebaut (Seite 31-32). Sie weisen jedoch eine große Empfindlichkeit gegenüber polaren Verbindungen wie beispielsweise Wasser, Alkoholen oder Ether auf. Colautti et al. in Chim. Ther. (1971), 6(5) 367-79 beschreiben die Herstellung von 4-Arylidenamino-4H-1,2,4-triazolen durch kondensierung von aromatischen Aldehyden und Ketonen mit 4-Amino-4H-1,2,4-Triazolen.

Gilyanovskii offenbart in Koord. Khim. (1985), 11, 889-895 die Darstellung Bis(salicylidenamino-1,2,4-triazol)-Komplexen von Cu, Ni, Co, Zn, Cd, Hg und Be. Bach et al. beschreiben in Z. Naturforsch., B, Chem. Sci. (1996), 51(6), 757-764 Metallkomplexe Bis- und Tris(N-(1-pyrrolyl)-salicylaldimin)-Komplexe von Cu, Co und Ni. Wang et al. offenbart in Yingyong Huaxue (1997), 14(3), 29-32 Cu-Komplexe von symmetrischen Triazol und Salicylamid.

In der WO 00/50470 werden polymerisationsaktive Nickel-Komplexe offenbart, die einen Pyrol-Substituenten am Aldimin-Stickstoffatom tragen.

In DE-A 199 61 340, wird gezeigt, dass Komplexe später Übergangsmetalle der allgemeinen Formeln C und D 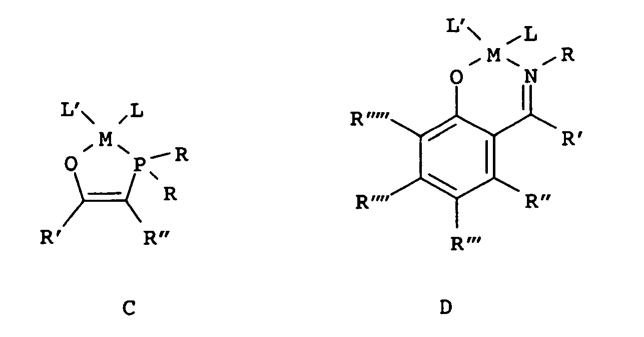 sowie Mischungen derselben bevorzugt eignen, um durch Emulsionspolymerisation Ethylen zu polymerisieren, wobei R bis R""' für Wasserstoff, Alkyl, C₇-C₁₃-Aralkyl oder C₆-C₁₄-Arylgruppen steht. Die Aktivitäten sollten sich jedoch noch verbessern lassen. In A. Held et al., J. Chem. Soc., Chem. Commun. 2000, 301 wird gezeigt, dass sich Komplexe der allgemeinen Formel C, bei denen R für Phenyl und R" für eine SO₃⁻-Gruppe steht, in wässrigem Medium Ethylen polymerisieren. Auch die Aktivität von C ist noch nicht optimal.

Aufgrund der großen kommerziellen Bedeutung von Polyolefinen ist die Suche nach möglichst vielseitigen polymerisationsaktiven Komplexen mit möglichst hoher Aktivität auch weiterhin von großer Bedeutung.

Speziell bestand die Aufgabe,
- neue Komplexverbindungen bereitzustellen, die zur Polymerisation von Olefinen geeignet sind;
- ein Verfahren zur Herstellung der polymerisationsaktiven Komplexverbindungen bereitzustellen;
- ein Verfahren zur Polymerisation oder Copolymerisation von Olefinen unter Verwendung der polymerisatakiven Komplexverbindungen bereitzustellen;
- Trägerkatalysatoren für die Polymerisation von Olefinen sowie ein Verfahren zur Herstellung der polymerisatakiven Trägerkatalysatoren unter Verwendung der polymerisatakiven Komplexverbindungen bereitzustellen;
- mit den polymerisatakiven Trägerkatalysatoren Olefine zu polymerisieren und zu copolymerisieren;
- ein Verfahren zur Emulsionspolymerisation oder Copolymerisation von Olefinen, insbesondere Ethylen, unter Verwendung der erfindungsgemäßen Komplexe bereitzustellen.

Überraschend wurde nun gefunden, dass die Aufgabe mit Hilfe von solchen Komplexverbindungen gelöst werden kann, die die eingangs definierten Strukturen der allgemeinen Formel I aufweisen.

In Formel I sind die Variablen wie folgt definiert:
- M: ein Element der 6. bis 10. Gruppe des Periodensystem der Elemente. Bevorzugt ist M ausgewählt aus Cr, Fe oder Pd..
- Nu¹: ist ausgewählt aus O, S und Se, wobei Sauerstoff bevorzugt ist;
- Nu², Nu³: gleich oder verschieden und ausgewählt aus N oder P, wobei Nu² = Nu³ = Stickstoff besonders bevorzugt ist;
- A¹: ist N oder C-R⁷ oder Si-R⁷, wobei N oder C-R⁷ bevorzugt sind, besonders bevorzugt ist C-R⁷;
- A²: ist N oder C-R⁸ oder Si-R⁸, wobei N oder C-R⁸ bevorzugt sind, besonders bevorzugt ist C-R⁸;
- R¹ bis R⁹: sind gleich oder verschieden und ausgewählt aus
- Wasserstoff,
- C₁-C₈-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl und n-Octyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
- Unter den substituierten C₁-C₈-Alkylgruppen seien beispielhaft genannt: ein- oder mehrfach halogenierte C₁-C₈-Alkylgruppen wie Fluormethyl, Difluormethyl, Trifluormethyl, Chlormethyl, Dichlormethyl, Trichlormethyl, Brommethyl, Dibrommethyl, Tribrommethyl, Pentafluorethyl, Perfluorpropyl und Perfluorbutyl, besonders bevorzugt sind Fluormethyl, Difluormethyl, Trifluormethyl und Perfluorbutyl;
- C₂-C₈-Alkenyl mit ein bis 4 isolierten oder konjugierten Doppelbindungen, beispielsweise Vinyl, 1-Allyl, 3-Allyl, ω-Butenyl, ω-Pentenyl, ω-Hexenyl, 1-cis-Buta-1,3-dienyl oder 1-cis
- Hexa-1,5-dienyl.
- Unter den substituierten C₂-C₈-Alkenylgruppen seien beispielhaft genannt: Isopropenyl, 1-Isoprenyl, α-Styryl, β-Styryl, 1-cis-1,2-Phenylethenyl oder 1-trans-1,2-Phenylethenyl.
- C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
- unter den substituierten Cycloalkylgruppen seien beispielhaft genannt: 2-Methylcyclopentyl, 3-Methylcyclopentyl, cis-2,4-Dimethylcyclopentyl, trans-2,4-Dimethylcyclopentyl, cis-2,5-Dimethylcyclopentyl, trans-2,5-Dimethylcyclopentvl, 2,2,5,5-Tetramethylcyclopentyl, 2-Methylcyclohexyl, 3-Methylcyclohexyl, 4-Methylcyclohexyl, cis-2,6-Dimethylcyclohexyl, trans-2,6-Dimethylcyclohexyl, cis-2,6-Diisopropylcyclohexyl, trans-2,6-Diisopropylcyclohexyl, 2,2,6,6-Tetramethylcyclohexyl, 2-Methoxycyclopentyl, 2-Methoxycyclohexyl, 3-Methoxycyclopentyl, 3-Methoxycyclohexyl, 2-Chlorcyclopentyl, 3-Chlorcyclopentyl, 2,4-Dichlorcyclopentyl, 2,2,4,4-Tetrachlorcyclopentyl, 2-Chlorcyclohexyl, 3-Chlorcyclohexyl, 4-Chlorcyclohexyl, 2,5-Dichlorcyclohexyl, 2,2,6,6-Tetrachlorcyclohexyl, 2-Thiomethylcyclopentyl, 2-Thiomethylcyclohexyl, 3-Thio-methylcyclopentyl, 3-Thiomethylcyclohexyl und weitere Derivate;
- C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl;
- C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
- C₆-C₁₄-Aryl wie Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, gleich oder verschieden substituiert durch eine oder mehrere
   - C₁-C₈-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl und n-Octyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
   - Unter den substituierten C₁-C₈-Alkylgruppen seien beispielhaft genannt: ein- oder mehrfach halogenierte C₁-C₈-Alkylgruppen wie Fluormethyl, Difluormethyl, Trifluormethyl, Chlormethyl, Dichlormethyl, Trichlormethyl, Brommethyl, Dibrommethyl, Tribrommethyl, Pentafluorethyl, Perfluorpropyl und Perfluorbutyl, besonders bevorzugt sind Fluormethyl, Difluormethyl, Trifluormethyl und Perfluorbutyl;
   - C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
   - C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl;
   - C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
   - Halogen, beispielsweise Fluor, Chlor, Brom oder Iod, besonders bevorzugt Fluor oder Chlor;
   - C₁-C₆-Alkoxygruppen wie Methoxy, Ethoxy, n-Propoxy, iso-Propoxy, n-Butoxy, iso-Butoxy, sec.-Butoxy, tert.-Butoxy, n-Pentoxy, iso-Pentoxy, n-Hexoxy und iso-Hexoxy, besonders bevorzugt Methoxy, Ethoxy, n-Propoxy und n-Butoxy;
   - C₆-C₁₄-Aryloxygruppen wie Phenoxy, ortho-Kresyloxy, meta-Kresyloxy, para-Kresyloxy, α-Naphthoxy, β-Naphthoxy oder 9-Anthryloxy;
   - Silylgruppen SiR¹⁰R¹¹R¹², wobei R¹⁰ bis R¹² unabhängig voneinander aus Wasserstoff, C₁-C₈-Alkylgruppen, Benzylresten und C₆-C₁₄-Arylgruppen ausgewählt sind; bevorzugt sind die Trimethylsilyl-, Triethylsilyl-, Triisopropylsilyl-, Diethylisopropylsilyl-, Dimethylthexylsilyl-, tert.-Butyldimethylsilyl-, tert.-Butyldiphenylsilyl-, Tribenzylsilyl-, Triphenylsilyl- und die Tri-para-xylylsilylgruppe; besonders bevorzugt sind die Trimethylsilylgruppe und die tert.-Butyldimethylsilylgruppe;
   - Silyloxygruppen OSiR¹⁰R¹¹R¹², wobei R¹⁰ bis R¹² unabhängig voneinander aus Wasserstoff, C₁-C₈-Alkylgruppen, Benzylresten und C₆-C₁₄-Arylgruppen ausgewählt sind; bevorzugt sind die Trimethylsilyloxy-, Triethylsilyloxy-, Triisopropylsilyloxy-, Diethylisopropylsilyloxy-, Dimethylthexylsilyloxy-, tert.-Butyldimethylsilyloxy-, tert.-Butyldiphenylsilyloxy-, Tribenzylsilyloxy-, Triphenylsilyloxy- und die Tri-para-xylylsilyloxygruppe; besonders bevorzugt sind die Trimethylsilyloxygruppe und die tert.-Butyldimethylsilyloxygruppe;
- fünf- bis sechsgliedrigen stickstoffhaltigen Heteroarylresten wie beispielsweise *N*-Pyrrolyl, Pyrrol-2-yl, Pyrrol-3-yl, *N-*Imidazolyl, 2-Imidazolyl, 4-Imidazolyl, 1,2,4-Triazol-3-yl, 1,2,4-Triazol-4-yl, 2-Pyridyl, 3-Pyridyl, 4-Pyridyl, 3-Pyridazinyl, 4-Pyridazinyl, 2-Pyrimidinyl, 4-Pyrimidinyl, 5-Pyrimidinyl, N-Indolyl und N-Carbazolyl;
- fünf- bis sechsgliedrigen stickstoffhaltigen Heteroarylresten wie beispielsweise *N*-Pyrrolyl, Pyrrol-2-yl, Pyrrol-3-yl, *N-*Imidazolyl, 2-Imidazolyl, 4-Imidazolyl, 1,2,4-Triazol-3-yl, 1,2,4-Triazol-4-yl, 2-Pyridyl, 3-Pyridyl, 4-Pyridyl, 3-Pyridazinyl, 4-Pyridazinyl, 2-Pyrimidinyl, 4-Pyrimidinyl, 5-Pyrimidinyl, N-Indolyl und N-Carbazolyl, gleich oder verschieden einfach oder mehrfach substituiert mit
   - C₁-C₈-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl und n-Octyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
   - Unter den substituierten C₁-C₈-Alkylgruppen seien beispielhaft genannt: ein- oder mehrfach halogenierte C₁-C₈-Alkylgruppen wie Fluormethyl, Difluormethyl, Trifluormethyl, Chlormethyl, Dichlormethyl, Trichlormethyl, Brommethyl, Dibrommethyl, Tribrommethyl, Pentafluorethyl, Perfluorpropyl und Perfluorbutyl, besonders bevorzugt sind Fluormethyl, Difluormethyl, Trifluormethyl und Perfluorbutyl;
   - C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
   - C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl;
   - C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
   - Halogen, beispielsweise Fluor, Chlor, Brom oder Iod, besonders bevorzugt Fluor oder Chlor;
   - C₁-C₆-Alkoxygruppen wie Methoxy, Ethoxy, n-Propoxy, iso-Propoxy, n-Butoxy, iso-Butoxy, sec.-Butoxy, tert.-Butoxy, n-Pentoxy, iso-Pentoxy, n-Hexoxy und iso-Hexoxy, besonders bevorzugt Methoxy, Ethoxy, n-Propoxy und n-Butoxy;
   - C₆-C₁₄-Aryloxygruppen wie Phenoxy, ortho-Kresyloxy, meta-Kresyloxy, para-Kresyloxy, α-Naphthoxy, β-Naphthoxy oder 9-Anthryloxy;
   - Silylgruppen SiR¹⁰R¹¹R¹², wobei R¹⁰ bis R¹² unabhängig voneinander aus Wasserstoff, C₁-C₈-Alkylgruppen, Benzylresten und C₆-C₁₄-Arylgruppen ausgewählt sind; bevorzugt sind die Trimethylsilyl-, Triethylsilyl-, Triisopropylsilyl-, Diethylisopropylsilyl-, Dimethylthexylsilyl-, tert.-Butyldimethylsilyl-, tert.-Butyldiphenylsilyl-, Tribenzylsilyl-, Triphenylsilyl- und die Tri-para-xylylsilylgruppe; besonders bevorzugt sind die Trimethylsilylgruppe und die tert.-Butyldimethylsilylgruppe;
   - Silyloxygruppen OSiR¹⁰R¹¹R¹², wobei R¹⁰ bis R¹² unabhängig voneinander aus Wasserstoff, C₁-C₈-Alkylgruppen, Benzylresten und C₆-C₁₄-Arylgruppen ausgewählt sind; bevorzugt sind die Trimethylsilyloxy-, Triethylsilyloxy-, Triisopropylsilyloxy-, Diethylisopropylsilyloxy-, Dimethylthexylsilyloxy-, tert.-Butyldimethylsilyloxy-, tert.-Butyldiphenylsilyloxy-, Tribenzylsilyloxy-, Triphenylsilyloxy- und die Tri-para-xylylsilyloxygruppe; besonders bevorzugt sind die Trimethylsilyloxygruppe und die tert.-Butyldimethylsilyloxygruppe;

In einer besonders bevorzugten Ausführungsform sind R¹, R² und R⁴ Wasserstoff. In einer ebenfalls besonders bevorzugten Ausführungsform sind R⁶ und R⁹ C₁-C₈-Alkyl, verzweigt oder unverzweigt. R³ und R⁵ sind besonders bevorzugt unabhängig voneinander Wasserstoff oder C₁-C₈-Alkyl, verzweigt oder unverzweigt.

In einer besonderen Ausführungsform können benachbarte Reste R¹ bis R⁹ miteinander zu einem 5 bis 12-gliedrigen Ring verbunden sein. Beispielsweise können R⁶ und R⁷ zusammen sein: -(CH₂)₃₋(Trimethylen), -(CH₂)₄- (Tetramethylen), -(CH₂)₅- (Pentamethylen), -(CH₂)₆- (Hexamethylen), -CH₂-CH=CH-, -CH₂-CH=CH-CH₂-, -CH=CH-CH=CH-, -O-CH₂-O-, -O-CHMe-O-, -O-CH-(C₆H₅)-O-, -O-CH₂-CH₂-O-, -O-CMe₂-O-, -NMe-CH₂-CH₂-NMe-, -NMe-CH₂-NMe- oder -O-SiMe₂-O- mit Me = CH₃. In einem bevorzugten Beispiel bilden R⁶ und R⁷ zusammen eine 1,3-Butadien-1,4-diyl-Einheit, die ihrerseits mit C₁-C₈-Alkyl ein oder mehrfach substituiert sein kann. In einem weiteren bevorzugten Beispiel bilden R⁶ und R⁷ sowie R⁸ und R⁹ jeweils paarweise zusammen eine 1,3-Butadien-1,4-diyl-Einheit, die ihrerseits mit C₁-C₈-Alkyl ein oder mehrfach substituiert sein können.
- L¹: wird gewählt anorganischen oder organischen Neutralliganden, beispielsweise aus Phosphanen der Formel (R¹³)ₓPH₃₋ₓ oder Aminen der Formel (R¹³)ₓNH₃₋ₓ, wobei x eine ganze Zahl von 0 und 3 bedeutet. Aber auch Ether (R¹³)₂O wie beispielsweise Dialkylether, z.B. Diethylether, oder cyclische Ether, wie beispielsweise Tetrahydrofuran, H₂O, Alkohole (R¹³)OH wie Methanol oder Ethanol, Pyridin, Pyridinderivate der Formel C₅H₅₋ₓ(R¹³)ₓN, wie beispielsweise 2-Picolin, 3-Picolin, 4-Picolin, 2,3-Lutidin, 2,4-Lutidin, 2,5-Lutidin, 2,6-Lutidin oder 3,5-Lutidin, CO, C₁-C₁₂-Alkylnitrile oder C₆-C₁₄-Arylnitrile sind geeignet, wie Acetonitril, Propionitril, Butyronitril oder Benzonitril. Weiterhin können einfach oder mehrfach ethylenisch ungesättigte Doppelbindungssysteme als Ligand dienen, wie Ethenyl, Propenyl, cis-2-Butenyl, trans-2-Butenyl, Cyclohexenyl oder Norbornenyl.
- L²: wird ausgewählt anorganischen oder organischen anionischen Liganden, beispielsweise aus
- Halogenidionen wie Fluorid, Chlorid, Bromid, oder Iodid, bevorzugt sind Chlorid und Bromid,
- Amidanionen (R¹³)ₓ₋₁NH₂₋ₓ, wobei x eine ganze Zahl von 0 bis 3 bedeutet,
- C₁-C₆-Alkylanionen wie (CH₃)⁻, (C₂H₅)⁻, (C₃H₇)⁻, (n-C₄H₉)⁻, (tert.-C₄H₉)⁻ oder (C₆H₁₄)⁻;
- Allylanionen oder Methallylanionen,
- Benzylanionen oder
- Arylanionen wie (C₆H₅)⁻.
- z: ist eine ganze Zahl von 1 bis 3, wie 0, 1, 2 oder 3;
- R¹³: sind gleich oder verschieden und ausgewählt aus
Wasserstoff,
C₁-C₈-Alkylgruppen,C₃-C₁₂-Cycloalkyl, C₇-C₁₃-Aralkyl
Benzylresten und
C₆-C₁₄-Arylgruppen, wobei diese Gruppen wie vorstehend definiert sind und wobei zwei Reste R¹³ miteinander kovalent verknüpft sein können,

In einer besonderen Ausführungsform sind L¹ und L² miteinander durch eine oder mehrere kovalente Bindungen verknüpft. Beispiele für solche Liganden sind 1,5-Cyclooctadienyl-Liganden ("COD"), 1,6-Cyclodecenyl-Liganden oder 1,5,9-*all-trans*-Cyclododecatrienyl-Liganden.

In einer weiteren besonderen Ausführung ist L¹ Tetramethylethylendiamin, wobei nur ein Stickstoff mit dem Nickel koordiniert.

Bevorzugt sind weiterhin Komplexverbindungen, bei denen die Variablen wie folgt definiert sind:
- M: ein Element der 6. bis 10. Gruppe des Periodensystems der Elemente, ausser Nickel
- Nu¹: Sauerstoff,
- Nu², Nu³: Stickstoff,
- A¹: N oder C-R⁸,
- A²: N oder C-R⁹,
- R¹: Wasserstoff,
C₁-C₈-Alkyl, substituiert oder unsubstituiert,
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit C₁-C₈-Alkyl, substituiert oder unsubstituiert,
- R² bis R⁹: gleich oder verschieden und ausgewählt aus Wasserstoff,
C₁-C₈-Alkyl, substituiert oder unsubstituiert,
C₂-C₈-Alkenyl, substituiert oder unsubstituiert, mit ein bis 4 isolierten oder konjugierten Doppelbindungen;
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit
C₁-C₈-Alkyl, substituiert oder unsubstituiert,
Halogen,
C₁-C₆-Alkoxy,
wobei räumlich benachbarte Reste R¹ bis R⁹ miteinander zu einem 5-bis 12-gliedrigen Ring verbunden sein können.

Die Herstellung der erfindungsgemäßen Komplexverbindungen der allgemeinen Formel I 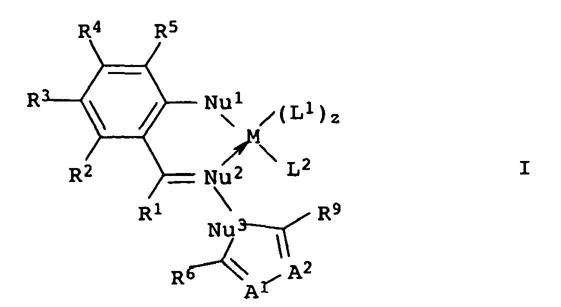 bei denen die Variablen wir folgt definiert sind:
- M: ein Element der 6. bis 10. Gruppe des Periodensystems der Elemente,
- Nu¹: ausgewählt aus O, S oder Se;
- Nu², Nu³: ausgewählt aus N oder P,
- A¹: N oder C-R⁷ oder Si-R⁷,
- A²: N oder C-R⁸ oder Si-R⁸,
- R¹ bis R⁹: ausgewählt aus
Wasserstoff,
C₁-C₈-Alkyl, substituiert oder unsubstituiert,
C₂-C₈-Alkenyl, substituiert oder unsubstituiert, mit ein bis 4 isolierten oder konjugierten Doppelbindungen;
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy, substituiert oder unsubstituiert,
C₆-C₁₄-Aryloxy,
SiR¹⁰R¹¹R¹² oder O-SiR¹⁰R¹¹R¹²;
fünf- bis sechsgliedrigen stickstoffhaltigen Heteroarylresten, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-A-ryl,
Halogen,
C₁-C₆-Alkoxy,
C₆-C₁₄-Aryloxy,
SiR¹⁰R¹¹R¹² oder O-SiR¹⁰R¹¹R¹²;
wobei räumlich benachbarte Reste R¹ bis R⁹ miteinander zu einem 5-bis 12-gliedrigen Ring verbunden sein können;
- L¹: ein organischer oder anorganischer Neutralligand,
- L²: ein organischer oder anorganischer anionischer Ligand, wobei L¹ und L² miteinander durch eine oder mehrere kovalente Bindungen verknüpft sein können,
- z: eine ganze Zahl von 1 bis 3,

R¹⁰ bis R¹² gleich oder verschieden und ausgewählt werden aus Wasserstoff, C₁-C₈-Alkyl, C₃-C₁₂-Cycloalkyl, C₇-C₁₃-Aralkyl oder C₆-C₁₄-Aryl,
erfolgt im Allgemeinen aus Liganden der allgemeinen Formel II 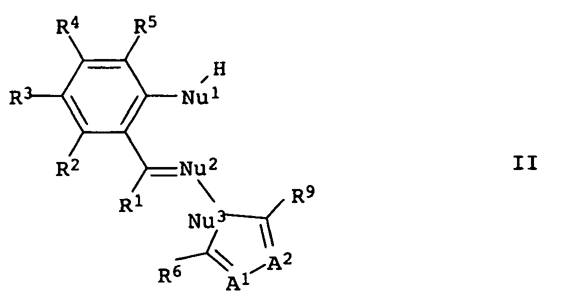 , in denen die Variablen wie oben definiert sind. Zur Synthese der erfindungsgemäßen Komplexverbindungen werden die Liganden zunächst mit Hilfe einer Base deprotoniert und anschließend mit Metallverbindungen der allgemeinen Formeln MX₂, MX₃, MX₄ oder ML¹L² umgesetzt, wobei MX₂, MX₃ oder MX₄ durch weitere Neutralliganden stabilisiert sind. Weiterhin bevorzugt ist das oben beschriebene Verfahren zur Herstellung von Komplexverbindungen der allgemeinen Formel I, wobei M ein Element der 6. bis 10. Gruppe des Periodensystems der Elemente, ausser Nickel ist und wobei MX₂, MX₃ oder MX₄ optional durch weitere Neutralliganden stabilisiert sind.

Als Base können die in der Metallorganischen Chemie gängigen Metallalkyle verwendet werden wie beispielsweise Methyllithium, Ethyllithium, n-Butyllithium, sec-Butyllithium, tert.-Butyllithium oder Hexyllithium, weiterhin Grignard-Verbindungen wie beispielsweise Ethylmagnesiumbromid, weiterhin Lithiumamid, Natriumamid, Kaliumamid, Kaliumhydrid oder Lithiumdiisopropylamid ("LDA"). Weiterhin bevorzugt ist das oben beschriebene Verfahren zur Herstellung von Komplexverbindungen der allgemeinen Formel I, wobei M ein Element der 6. bis 10. Gruppe des Periodensystems der Elemente ist und wobei MX₂, MX₃ oder MX₄ optional durch weitere Neutralliganden stabilisiert sind und wobei als Base Methyllithium, Ethyllithium, n-Butyllithium, sec-Butyllithium, tert.-Butyllithium oder Hexyllithium, Grignard-Verbindungen, Lithiumamid, Kaliumamid, Kaliumhydrid oder Lithiumdiisopropylamid verwendet werden. Als Lösemittel haben sich hochsiedende Lösemittel wie Toluol, ortho-Xylol, meta-Xylol, para-Xylol, Ethylbenzol oder Mischungen derselben als geeignet erwiesen, des Weiteren nichtcyclische oder cyclische Ether wie 1,2-Dimethoxyethan, Tetrahydrofuran oder Diethylether.

Diese Deprotonierung ist im Allgemeinen nach einigen Stunden beendet, sinnvoll ist eine Reaktionsdauer von 2 bis 10 Stunden, bevorzugt sind 3 bis 5 Stunden. Die Temperaturbedingungen sind im Allgemeinen unkritisch, eine Durchführung bei Temperaturen von -90°C bis -20°C hat sich als bevorzugt erwiesen.

Der deprotonierte Ligand und die Metallverbindung der allgemeinen Formeln MX₂, MX₃, MX₄ oder ML¹L² werden anschließend miteinander umgesetzt.
- X: sind gleich oder verschieden und gewählt aus:

Halogen, wie Fluor, Chlor, Brom oder Iod, bevorzugt sind Chlor und Brom;
- C₁-C₈-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl und n-Octyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
- C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
- C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl;
- C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
bevorzugt sind X gleich.

Dabei können MX₂, MX₃, MX₄ oder ML¹L² optional durch Neutralliganden stabilisiert werden. Als Neutralliganden bieten sich die gängigen Liganden der Komplexchemie an, wie beispielsweise cyclische und nichtcyclische Ether, Amine, Diamine, Nitrile, Isonitrile oder Phosphine. Besonders bevorzugt sind Diethylether, Tetrahydrofuran, 1,2-Dimethoxyethan, Tetramethylethylendiamin, Acetonitril oder Triphenylphosphan. Insbesondere in Fällen, in denen beispielsweise Ni-Dialkylverbindungen eingesetzt werden sollen, haben sich Neutralliganden bewährt. Die Neutralliganden können auch als Lösemittel verwendet werden.

Die Bedingungen für die Umsetzung sind an sich unkritisch; üblicherweise mischt man den deprotonierten Liganden II und MX₂ oder MX₄ miteinander in einem geeigneten Lösemittel wie Benzol, Toluol, Ethylbenzol, ortho-Xylol, meta-Xylol oder para-Xylol, Chlorbenzol, Cyclohexan, Acetonitril, Tetrahydrofuran, Methylenchlorid oder Gemischen derselben. Als Temperaturbereich kommen -100°C bis +150°C in Frage, bevorzugt -78°C bis +100°C. Wichtig ist, dass man die Umsetzung unter Ausschluss von Sauerstoff und Feuchtigkeit durchführt.

Als Molverhältnisse zwischen Ligand und M sind solche im Bereich von 5:1 bis 1:5'geeignet. Da jedoch die erfindungsgemäßen Liganden der allgemeinen Formel II die aufwändiger zugänglichen Reaktionspartner sind, sind Molverhältnisse Ligand : M im Bereich von 1:1 bis 1:3 bevorzugt, besonders bevorzugt sind stöchiometrische Mengen.

Die Reinigung der erfindungsgemäßen Komplexverbindungen der allgemeinen Formel I gelingt durch die in der metallorganischen Chemie üblichen Methoden, wobei die Kristallisation besonders bevorzugt ist, weiterhin sind Filtrationen über Filterhilfsmittel wie beispielsweise Celite® geeignet.

Für die Polymerisation ist es nicht in allen Fällen notwendig, die erfindungsgemäßen Komplexverbindungen zu isolieren. Mann kann auch einen Liganden der allgemeinen Formel II mit einer geeigneten Metallverbindung der Formel MX₂, MX₃, MX₄ oder ML¹L² erst unmittelbar vor der Polymerisation miteinander umsetzen und *in situ* erzeugen.

Wählt man X in der Metallverbindung der Formel MX₂, MX₃, oder MX₄ oder L² in ML¹L² aus der Gruppe der C₁-C₆-Alkylgruppen, Benzylanionen oder Arylanionen aus, so kann man auf die Deprotonierung des Liganden der allgemeinen Formel II verzichten. In diesen Fällen hat sich als bevorzugt erwiesen, die erfindungsgemäßen Komplexverbindungen nicht zu isolieren, sondern erst unmittelbar vor der Polymerisation *in situ* zu erzeugen.

Die Herstellung der erfindungsgemäßen Liganden der allgemeinen Formel II gelingt durch Kondensation einer Carbonylverbindung der allgemeinen Formel III 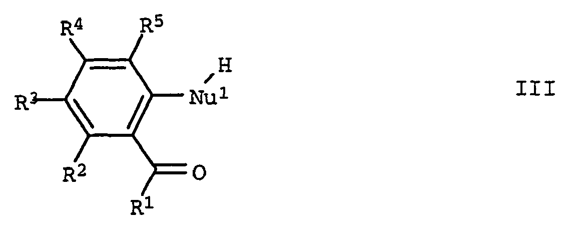 mit einer Verbindung der allgemeinen Formel IV, 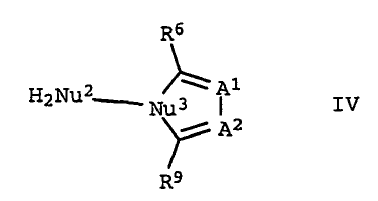 in denen die Variablen wie eingangs definiert sind.

Die Synthese der Liganden der allgemeinen Formel II wird bei Temperaturen von -78°C bis + 150°C durchgeführt, bevorzugt bei -10°C bis +75°C. Als Katalysator wird eine Lewis-Säure oder eine Brønsted-Säure zugegeben. Als Lewis-Säuren haben sich insbesondere Aluminiumalkyle wie Al(CH₃)₃, Al(C₂H₅)₃ oder BF₃ als wirksam erwiesen. Als Brønsted-Säuren lassen sich beispielsweise Schwefelsäure, Phosphorsäure, HF, Toluolsulfonsäure oder Amidosulfonsäure verwenden. Die Reaktionsdauer beträgt 1 bis 48 Stunden, bevorzugt 12 bis 24 Stunden. Als Lösemittel haben sich aprotische Medien als bevorzugt erwiesen, insbesondere Toluol und Benzol, wenn Aluminiumalkyle oder BF₃ als Katalysatoren verwendet wurden, ansonsten lassen sich auch Alkohole wie Methanol, Ethanol oder Mischungen derselben verwenden. Es hat sich - insbesondere bei der Verwendung einer Brønsted-Säure als Katalysator - als wirksam erwiesen, mit Hilfe eines Wasserabscheiders das gebildete Wasser azeotrop abzudestillieren.

Verbindungen der allgemeinen Formel III und ein Verfahren zu ihrer Herstellung sind in WO 98/42664 beschrieben.

Die Synthese der Verbindungen der allgemeinen Formel IV gelingt nach dem in DE-A 199 44 993, beschriebenen Verfahren, indem man zunächst eine geeignete 1,4-Dicarbonylverbindung der allgemeinen Formel V 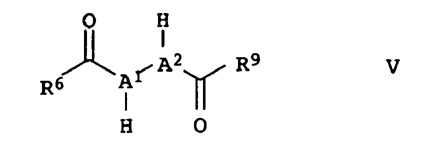 mit einem Äquivalent Acetylhydrazin oder Benzoylhydrazin in Anwesenheit einer katalytischen Menge Säure, bevorzugt para-Toluolsulfonsäure, in einem inerten Lösemittel, bevorzugt Toluol, zu einem N-Acetyl- oder N-Benzoyl-geschützten Derivat von IV umsetzt und anschließend mit einem Überschuß an Base, vorzugsweise KOH, in einem hochsiedenden organischen Lösemittel wie beispielsweise Ethylenglykol verseift.

Damit die erfindungsgemäßen Komplexe der allgemeinen Formeln I katalytisch aktiv sind, müssen sie aktiviert werden. Geeignete Aktivatoren sind ausgewählte Aluminium- oder Bor-Verbindungen mit elektronenziehenden Resten (z.B. Trispentafluorphenylboran, Trispentafluorphenylaluminium, *N,N*-Dimethylanilinium-tetrakis-pentafluorphenylborat, Tri-n-butylammonium-tetrakis-pentafluorphenylborat, *N,N*-Dimethylanilinium-tetrakis-(3,5-bisperfluormethyl)-phenylborat, Tri-n-butylammonium-tetrakis-(3,5-bisperfluormethyl)-phenylborat sowie Tritylium-tetrakispentafluorphenylborat). Bevorzugt sind Dimethylanilinium-tetrakis-pentafluorphenylborat, Tritylium-tetrakispentafluorphenylborat sowie Trispentafluorphenylboran.

Verwendet man Bor- oder Aluminiumverbindungen als Aktivatoren für die erfindungsgemäßen Komplexverbindungen der allgemeinen Formel I, so setzt man sie im Allgemeinen in einem molaren Verhältnis von 1:10 bis 10:1, bezogen auf M, ein; bevorzugt 1:2 bis 5:1 und besonders bevorzugt 1:1,5 bis 1,5:1.

Eine andere geeignete Klasse von Aktivatoren sind Aluminoxane.

Die Struktur der Aluminoxane ist nicht genau bekannt. Es handelt sich bei ihnen um Produkte, die durch vorsichtige partielle Hydrolyse von Aluminiumalkylen erhalten werden (s. DE-A 30 07 725). Diese Produkte liegen nicht rein vor, sondern als Gemische von offenkettigen und cyclischen Strukturen des Typs VI a und VI b. Diese Gemische liegen vermutlich in einem dynamischen Gleichgewicht zueinander vor.

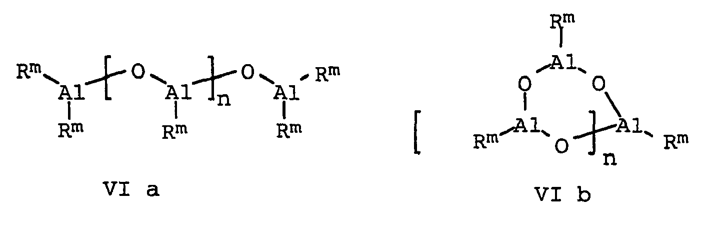

In Formel VI a und VI b sind die Reste R^{m} unabhängig voneinander
- C₁-C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt ist Methyl;
- C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, oder
- C₆-C₁₄-Aryl wie Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl; und

- n: ist eine ganze Zahl von 0 bis 40, bevorzugt von 1 bis 25 und besonders bevorzugt von 2 bis 22.

In der Literatur werden auch käfigartige Strukturen für Aluminoxane diskutiert (Y. Koide, S.G. Bott, A.R. Barron Organometallics 1996, 15, 2213-26; A.R. Barron Macromol. Symp. 1995, 97, 15-25). Unabhängig davon, wie die Struktur der Aluminoxane tatsächlich aussieht, sind sie als Aktivatoren der erfindungsgemäßen Metallkomplexe der allgemeinen Formel I geeignet.

Gemische verschiedener Aluminoxane sind in den Fällen besonders bevorzugte Aktivatoren, in denen in einer Lösung eines Paraffins, beispielsweise n-Heptan oder Isododekan, polymerisiert wird. Eine besonders bevorzugtes Gemisch ist das kommerziell bei der Firma Witco GmbH erhältliche CoMAO mit einer Formel von [(CH₃)_{0,9}(iso-C₄H₉)_{0,1}AlO]ₙ.

Um die erfingungsgemäßen Komplexverbindungen der allgemeinen Formel I mit Aluminoxanen zu aktivieren, ist im Allgemeinen ein Überschuss von Aluminoxan, bezogen auf M, notwendig. Sinnvolle Molverhältnisse M:AI liegen im Bereich von 1:10 bis 1:10 000, bevorzugt 1:50 bis 1:1000 und besonders bevorzugt 1:100 bis 1:500.

Aktivatoren für Metallkomplexe der allgemeinen Formel I abstrahieren nach gängiger Vorstellung einen Liganden L¹ oder L². Bei dem Aktivator kann es sich anstatt um Aluminiumalkylverbindungen der allgemeinen Formel VI a oder VI b oder den vorstehend beschriebenen Aluminium- oder Bor-Verbindungen mit elektronenziehenden Resten beispielsweise um Olefinkomplexe des Rhodiums oder Nickels handeln.

Bevorzugte, kommerziell bei Aldrich erhältliche Nickel-(Olefin)y-Komplexe mit y = 1, 2, 3 oder 4 sind Ni(C₂H₄)₃, Ni(1,5-Cyclooctadien)₂ "Ni(COD)₂", Ni(1,6-Cyclodecadien)₂, oder Ni(1,5,9-all-trans-Cyclododecatrien)₂. Besonders bevorzugt ist Ni(COD)₂.

Besonders geeignet sind gemischte Ethylen/1,3-Dicarbonylkomplexe des Rhodiums, beispielsweise Rhodium-Acetylacetonat-Ethylen Rh(acac) (CH₂=CH₂)₂, Rhodium-Benzoylacetonat-Ethylen Rh(C₆H₅-CO-CH-CO-CH₃) (CH₂=CH₂)₂ oder Rh(C₆H₅-CO-CH=CO-C₆H₅) (CH₂=CH₂)₂. Am besten geeignet ist Rh(acac)(CH₂=CH₂)₂. Diese Verbindung lässt sich nach den Angaben von R. Cramer aus Inorg. Synth. 1974, 15, 14 synthetisieren.

Einige Komplexe der allgemeinen Formel I lassen sich durch Ethylen aktivieren. Die Leichtigkeit der Aktivierungsreaktion hängt entscheidend von der Natur des Liganden L¹ ab.

Der gewählte Komplex der allgemeinen Formeln I und der Aktivator bilden zusammen ein Katalysatorsystem.

Durch Zugabe von weiterem Aluminiumalkyl der allgemeinen Formel Al(R^{m})₃ oder Aluminoxanen kann die Aktivität des erfindungsgemäßen Katalysatorsystems erhöht werden, insbesondere dann, wenn Verbindungen der allgemeinen Formel VI a oder VI b oder die vorstehend genannten Aluminium- oder Bor-Verbindungen mit elektronenziehenden Resten als Aktivatoren verwendet werden; Aluminiumalkyle der allgemeinen Formel Al(R^{m})₃ oder Aluminoxane können auch als Molmassenregler wirken. Ein weiterer effektiver Molmassenregler ist Wasserstoff. Besonders gut kann man die Molmasse durch die Reaktionstemperatur und den Druck regeln. Für den Fall, dass die Verwendung einer Bor-Verbindung wie oben beschrieben gewünscht ist, ist die Zugabe eines Aluminiumalkyls der allgemeinen Formel Al(R^{m})₃ besonders bevorzugt.

Es wurde gefunden, dass die erfindungsgemäßen Komplexe der allgemeinen Formel I geeignet sind, um Olefine zu polymerisieren. Besonders gut polymerisieren und copolymerisieren sie Ethylen und Propylen.

Druck- und Temperaturbedingungen während der Polymerisation können in weiten Grenzen gewählt werden. Als Druck hat sich ein Bereich von 0,05 MPa bis 400 MPa als geeignet erwiesen, bevorzugt sind 1 bis 7,5 MPa oder Hochdruckbedingungen von 50 bis 250 MPa. Als Temperatur hat sich ein Bereich von 0 bis 120°C als geeignet erwiesen, bevorzugt sind 40 bis 100°C und besonders bevorzugt 50 bis 85°C.

Als Monomer sind die folgenden Olefine zu nennen: Ethylen, Propylen, 1-Buten, 1-Penten, 1-Hexen, 1-Octen, 1-Decen oder 1-Undecen, wobei Propylen und Ethylen bevorzugt und Ethylen besonders bevorzugt ist.

Als Comonomere sind α-Olefine geeignet, wie beispielsweise 0,1 bis 20 mol-% 1-Buten, 1-Penten, 1-Hexen, 4-Methyl-1-Penten, 1-Octen, 1-Decen oder 1-Undecen. Aber auch Isobuten und Styrol sind geeignete Comonomere, des weiteren interne Olefine wie beispielsweise Cyclopenten, Cyclohexen, Norbornen und Norbornadien.

Als Lösemittel haben sich Toluol, ortho-Xylol, meta-Xylol, para-Xylol oder Ethylbenzol als geeignet erwiesen sowie Mischungen derselben, weiterhin Diethylether, Tetrahydrofuran, Chlorbenzol, 1,3-Dichlorbenzol, Dichlormethan und - bei Hochdruckbedingungen - überkritisches Ethylen.

Die erfindungsgemäßen Komplexverbindungen der allgemeinen Formel I lassen sich bei der Polymerisation mit Wasserstoff regeln, d.h. durch Zugabe von Wasserstoff lässt sich das Molekulargewicht der durch das erfindungsgemäße Katalysatorsystem erhältlichen Polymere senken. Bei genügend Wasserstoffzugabe werden Wachse erhalten, wobei die erforderliche Wasserstoffkonzentration auch von der Art der verwendeten Polymerisationsanlage abhängt.

Damit die erfindungsgemäßen Komplexverbindungen der allgemeinen Formel I in modernen Polymerisationsverfahren wie Suspensionsverfahren, Massepolymerisationsverfahren oder Gasphasenverfahren eingesetzt werden können, ist es notwendig, sie auf einem festen Träger zu immobilisieren. Andernfalls kann es zu Morphologieproblemen des Polymers (Brocken, Wandbeläge, Verstopfungen in Leitungen oder Wärmetauschern) kommen, die zum Abschalten der Anlage zwingen. Eine solche immobilisierte Komplexverbindung der allgemeinen Formel I wird als Katalysator bezeichnet.

Es wurde gefunden, dass sich die erfindungsgemäßen Komplexverbindungen der allgemeinen Formel I gut auf einem festen Träger abscheiden lassen. Als Trägermaterialien kommen z.B. poröse Metalloxide von Metallen der Gruppen 2 bis 14 oder Mischungen derselben in Frage, weiterhin Schichtsilikate und Zeolithe. Bevorzugte Beispiele für Metalloxide der Gruppen 2 bis 14 sind SiO₂, B₂O₃, Al₂O₃, MgO, CaO und ZnO. Bevorzugte Schichtsilikate sind Montmorrilonite oder Bentonite; als bevorzugter Zeolith wird MCM-41 eingesetzt.

Besonders bevorzugte Trägermaterialien sind sphärische Kieselgele und Alumosilikatgele der allgemeinen Formel SiO₂·a Al₂O₃, wobei a allgemein für eine Zahl im Bereich von 0 bis 2 steht, bevorzugt 0 bis 0,5. Derartige Kieselgele sind im Handel erhältlich, z.B. Silica Gel SG 332, Sylopol® 948 oder 952 oder S 2101 der Fa. W.R. Grace oder ES 70X der Fa. Crosfield.

Als Partikelgröße des Trägermaterials haben sich mittlere Teilchendurchmesser von 1 bis 300 µm bewährt, bevorzugt von 20 bis 80 µm, wobei der Teilchendurchmesser durch bekannte Methoden wie Siebmethoden bestimmt wird. Das Porenvolumen dieser Träger beträgt 1,0 bis 3,0 ml/g, bevorzugt von 1;6 bis 2,2 ml/g und besonders bevorzugt von 1,7 bis 1,9 ml/g. Die BET-Oberfläche beträgt 200 bis 750 m²/g, bevorzugt 250 bis 400 m²/g.

Um dem Trägermaterial anhaftende Verunreinigungen, insbesondere Feuchtigkeit, zu entfernen, können die Trägermaterialien vor der Dotierung ausgeheizt werden, wobei sich Temperaturen von 45 bis 1000°C eignen. Temperaturen von 100 bis 750°C sind für Kieselgele und andere Metalloxide besonders geeignet. Dieses Ausheizen kann über einen Zeitraum von 0,5 bis 24 Stunden erfolgen, wobei Ausheizzeiten von 1 bis 12 Stunden bevorzugt sind. Die Druckbedingungen sind vom gewählten Verfahren abhängig; das Ausheizen kann in einem Festbettverfahren, einem gerührten Kessel oder aber in einem Fließbettverfahren erfolgen. Ganz allgemein kann das Ausheizen bei Atmosphärendruck erfolgen. Vorteilhaft sind jedoch verminderte Drücke von 0,01 bis 50 kPa, besonders vorteilhaft ist ein Bereich von 0,1 bis 10 kPa und ganz besonders vorteilhaft ein Bereich von 0,2 bis 2 kPa. Für Fließbettverfahren hingegen empfiehlt es sich, bei leicht erhöhtem Druck zu arbeiten, wobei der Druck in einem Bereich von 101 kPa bis 500 kPa, bevorzugt 110 bis 150 kPa gewählt wird.

Eine chemische Vorbehandlung des Trägermaterials mit einer Alkylverbindung wie Aluminiumalkyl, Lithiumalkyl oder einem Aluminoxan ist ebenfalls möglich.

Für eine Polymerisation im Suspensionsverfahren werden solche Suspensionsmittel verwendet, in denen das gewünschte Polymer nicht oder nur in geringem Ausmaß löslich ist, weil andernfalls in den Anlagenteilen, in denen das Produkt vom Suspensionsmittel abgetrennt wird, Beläge des Produkts auftreten und zu wiederholten Abschaltungen und Reinigungsoperationen zwingen. Geeignete Suspensionsmittel sind gesättigte Kohlenwasserstoffe wie beispielsweise Propan, n-Butan, Isobutan, n-Pentan, Isopentan, n-Hexan, Isohexan und Cyclohexan, wobei Isobutan bevorzugt ist.

Druck- und Temperaturbedingungen während der Polymerisation können in weiten Grenzen gewählt werden. Als Druck hat sich ein Bereich von 0,05 MPa bis 15 MPa als geeignet erwiesen, bevorzugt sind 1 bis 7,5 MPa. Als Temperatur hat sich ein Bereich von 0 bis 120°C als geeignet erwiesen, bevorzugt sind 40 bis 100°C.

Als Monomer sind die folgenden Olefine zu nennen: Ethylen, Propylen, 1-Buten, 1-Penten, 1-Hexen, 1-Octen, 1-Decen oder 1-Undecen, wobei Ethylen bevorzugt ist.

Als Comonomere sind α-Olefine geeignet, wie beispielsweise 0,1 bis 20 mol-% 1-Buten, 1-Penten, 1-Hexen, 4-Methyl-1-Penten, 1-Octen, 1-Decen oder 1-Undecen. Aber auch Isobuten und Styrol sind geeignete Comonomere, des weiteren interne Olefine wie beispielsweise Cyclopenten, Cyclohexen, Norbornen und Norbornadien.

Die erfindungsgemäßen Katalysatoren haben sich weiterhin als Wasserstoff-regelbar erwiesen, d.h. durch Zugabe von Wasserstoff lässt sich das Molekulargewicht der durch die erfindungsgemäßen Katalysatoren erhältlichen Polymere senken. Bei genügend Wasserstoffzugabe werden Wachse erhalten, wobei die erforderliche Wasserstoffkonzentration auch von der Art der verwendeten Polymerisationsanlage abhängt. Bei Wasserstoffzugabe steigt die Aktivität der erfindungsgemäßen Katalysatoren.

Die erfindungsgemäßen Katalysatoren können auch gemeinsam mit einem oder mehreren anderen, an sich bekannten Polymerisationskatalysatoren verwendet werden. So können sie zusammen mit
- Ziegler-Natta-Katalysatoren,
- geträgerten Metallocenkatalysatoren der Übergangsmetalle der Gruppen 4 bis 6 des Periodensystems der Elemente,
- Katalysatoren der späten Übergangsmetalle (WO 96/23010),
- Fe- oder Co-Komplexen mit Pyridyldiiminliganden, wie sie in WO 98/27124 offenbart werden,
- oder auch Chromoxidkatalysatoren nach Phillips eingesetzt werden.

Dabei ist es einerseits möglich, verschiedene Katalysatoren miteinander zu mischen und gemeinsam zu dosieren oder cogeträgerte Komplexe auf einem gemeinsamen Träger zu verwenden oder auch verschiedene Katalysatoren getrennt an derselben oder an verschiedenen Stellen in das Polymerisationsgefäß zu dosieren.

Es wurde außerdem gefunden, dass sich die erfindungsgemäßen Komplexe der allgemeinen Formel I, insbesondere solche mit M = Ni, in besonderer Weise zur Polymerisation oder Copolymerisation von 1-Olefinen, bevorzugt Ethylen, in Emulsionspolymerisationsverfahren eignen.

Neben anderen 1-Olefinen als Comonomeren, wie beispielsweise Propen, 1-Buten, 1-Hexen, 1-Octen oder 1-Decen, lassen sich mit Hilfe des erfindungsgemäßen Katalysatorsystems auch polare Comonomere einbauen, wobei 0,1 bis 50 mol-% Comonomer verwendet werden können. Bevorzugt sind
- Acrylate wie Acrylsäure, Acrylsäuremethylester, Acrylsäureethylester, Acrylsäure-(2-ethyl)-hexylester, Acrylsäure-n-butylester oder Acrylsäure-tert.-butylester;
- Methacrylsäure, Methacrylsäuremethylester, Methacrylsäureethylester, Methacrylsäure-n-butylester oder Methacrylsäuretert.-butylester;
- Vinylcarboxylate, wobei Vinylacetat besonders bevorzugt ist,
- Ungesättigte Dicarbonsäuren, besonders bevorzugt ist Maleinsäure,
- ungesättigte Dicarbonsäurederivate, besonders bevorzugt sind Maleinsäureanhydrid und Maleinsäurealkylimide wie beispielsweise Maleinsäuremethylimid.

Weiterhin lassen sich Terpolymere mit mindestens 2 der oben aufgeführten Monomeren sowie Ethylen herstellen.

Die Emulsionspolymerisation der 1-Olefine unter Verwendung der erfindungsgemäßen Metallkomplexe der allgemeinen Formel I kann in an sich bekannter Weise durchgeführt werden.

Dabei ist die Reihenfolge der Zugabe der Reagenzien bei der Polymerisation unkritisch. So kann zunächst gasförmiges Monomer auf das Lösemittel aufgepresst bzw. flüssiges Monomer dosiert werden, und anschließend wird das Katalysatorsystem zugegeben. Man kann aber auch die Lösung des Katalysatorsystems zunächst mit weiterem Lösemittel verdünnen und anschließend Monomer zugeben.

Die eigentliche Polymerisation läuft üblicherweise bei einem Mindestdruck von 100 kPa, unterhalb dieses Druckes ist die Polymerisationsgeschwindigkeit zu gering. Bevorzugt sind 200 kPa und besonders bevorzugt ist ein Mindestdruck von 1000 kPa.

Als maximaler Druck sind 400 MPa zu nennen; bei höheren Drücken sind die Anforderungen an das Material des Polymerisationsreaktors sehr hoch, und der Prozess wird unwirtschaftlich. Bevorzugt sind 10 MPa und besonders bevorzugt sind 5 MPa.

Die Polymerisationstemperatur lässt sich in einem weiten Bereich variieren. Als Mindesttemperatur sind 10°C zu nennen, da bei tiefen Temperaturen die Polymerisationsgeschwindigkeit zurückgeht.

Bevorzugt ist eine Mindesttemperatur von 40°C und besonders bevorzugt sind 65°C. Als maximale sinnvolle Temperatur sind 350°C zu nennen und bevorzugt 150°C, besonders bevorzugt sind 100°C.

Vor der Polymerisation wird die Komplexverbindung der allgemeinen Formel I in einem organischen Lösemittel oder in Wasser gelöst. Durch mehrminütiges Rühren oder Schütteln wird gewährleistet, dass die Lösung klar ist. Dabei kann - je nach Löslichkeit der betreffenden Struktur - die Rührzeit zwischen 1 und 100 Minuten betragen.

Gleichzeitig wird der Aktivator, sofern er notwendig ist, in einer zweiten Portion desselben Lösemittels oder aber in Aceton gelöst.

Als organische Lösemittel eignen sich aromatische Lösemittel wie Benzol, Toluol, Ethylbenzol, ortho-Xylol, meta-Xylol und para-Xylol sowie Mischungen derselben. Des weiteren eignen sich cyclische Ether wie Tetrahydrofuran und Dioxan oder nicht cyclische Ether wie Diethylether, Di-*n*-butylether, Diisopropylether oder 1,2-Dimethoxyethan. Auch Ketone wie Aceton, Methylethylketon oder Diisobutylketon sind geeignet, desgleichen Amide wie Dimethylformamid oder Dimethylacetamid. Weiterhin sind Gemische dieser Lösemittel untereinander geeignet sowie Gemische dieser Lösemittel mit Wasser oder Alkoholen wie Methanol oder Ethanol.

Bevorzugt sind Aceton und Wasser sowie Mischungen aus Aceton und Wasser, wobei das Mischungsverhältnis beliebig ist. Die Menge des Lösemittels ist ebenfalls unkritisch, es muss jedoch gewährleistet sein, dass sich der Komplex und der Aktivator vollständig lösen können, andernfalls ist mit Aktivitätseinbußen zu rechnen. Der Lösungsvorgang kann gegebenenfalls durch Ultraschallbehandlung beschleunigt werden.

Ein optional zuzugebender Emulgator kann in einer dritten Portion des Lösemittels oder auch zusammen mit dem Komplex gelöst werden.

Dabei wird die Menge des Emulgators so gewählt, dass das Massenverhältnis zwischen Monomer und Emulgator größer als 1 ist, bevorzugt größer als 10 und besonders bevorzugt größer als 20. Dabei ist es umso günstiger, je weniger Emulgator verwendet werden muss. Die Aktivität in der Polymerisation wird deutlich gesteigert, wenn ein Emulgator zugegeben wird. Dieser Emulgator kann nichtionischer oder ionischer Natur sein.

Gebräuchliche nichtionische Emulgatoren sind z.B. ethoxylierte Mono-, Di- und Tri-Alkylphenole (EO-Grad: 3 bis 50, Alkylrest: C₄-C₁₂) sowie ethoxylierte Fettalkohole (EO-Grad: 3 bis 80; Alkylrest: C₈-C₃₆). Beispiele hierfür sind die Lutensol® -Marken der BASF AG oder die Triton® -Marken der Union Carbide.

Übliche anionische Emulgatoren sind z.B. Alkalimetall- und Ammoniumsalze von Alkylsulfaten (Alkylrest: C₈ bis C₁₂), von Schwefelsäurehalbestern ethoxylierter Alkanole (EO-Grad: 4 bis 30, Alkylrest: C₁₂-C₁₈) und ethoxylierter Alkylphenole (EO-Grad: 3 bis 50, Alkylrest: C₄-C₁₂), von Alkylsulfonsäuren (Alkylrest: C₁₂-C₁₈) und von Alkylarylsulfonsäuren (Alkylrest: C₉-C₁₈).

Geeignete kationische Emulgatoren sind in der Regel einen C₆-C₁₈-Alkyl-, -Aralkyl- oder heterocyclischen Rest aufweisende primäre, sekundäre, tertiäre oder quartäre Ammoniumsalze, Alkanolammoniumsalze, Pyridiniumsalze, Imidazoliniumsalze, Oxazoliniumsalze, Morpholiniumsalze, Thiazoliniumsalze sowie Salze von Aminoxiden, Chinoliniumsalze, Isochinoliniumsalze, Tropyliumsalze, Sulfoniumsalze und Phosphoniumsalze. Beispielhaft genannt seien Dodecylammoniumacetat oder das entsprechende Hydrochlorid, die Chloride oder Acetate der verschiedenen 2-(N,N,N-Trimethylammonium)ethylparaffinsäureester, N-Cetylpyridiniumchlorid, N-Laurylpyridiniumsulfat sowie N-Cetyl-N,N,N-trimethylammoniumbromid, N-Dodecyl-N,N,N-trimethylammoniumbromid, *N*,*N*-Distearyl-*N*,*N*-dimethylammoniumchlorid sowie das Gemini-Tensid N,N'-(Lauryldimethyl)ethylendiamindibromid. Zahlreiche weitere Beispiele finden sich in H. Stache, Tensid-Taschenbuch, Carl-Hanser-Verlag, München, Wien, 1981 und in McCutcheon's, Emulsifiers & Detergents, MC Publishing Company, Glen Rock, 1989.

Anschließend werden die Komponenten - Komplexverbindung in Lösung, optional die Lösung des Emulgators und optional die Lösung des Aktivators in den Polymerisationsreaktor gegeben. Als Polymerisationsreaktor haben sich gerührte Kessel und Autoklaven sowie Rohrreaktoren als brauchbar erwiesen, wobei die Rohrreaktoren als Schlaufenreaktor ausgeführt werden können.

Das oder die zu polymerisierenden Monomere werden in dem Polymerisationsmedium gemischt. Dabei können als Polymerisationsmedium Wasser oder Gemische von Wasser mit den oben aufgeführten Lösemitteln verwendet werden. Es ist zu beachten, dass der Anteil an Wasser mindestens 50 Vol.-% beträgt, bezogen auf die Gesamtmischung, bevorzugt mindestens 90 Vol.-% und besonders bevorzugt mindestens 95 Vol.-%.

Die Lösungen der Komplexverbindung, gegebenenfalls des Aktivators und gegebenenfalls des Emulgators werden mit dem Gemisch aus Monomer und wässrigem Polymerisationsmedium vereinigt. Die Reihenfolge der Zugabe der verschiedenen Komponenten ist an sich unkritisch. Es ist jedoch erforderlich, dass die Vereinigung der Komponenten so schnell erfolgt, dass keine Kristallisation von intermediär eventuell auftretenden schwer löslichen Komplexverbindungen erfolgt.

Das erfindungsgemäße Verfahren liefert Polyolefine und Olefincopolymere in hohen Ausbeuten, d.h. die Aktivität der erfindungsgemäßen Komplexe unter den Bedingungen der Emulsionspolymerisation ist sehr hoch.

Als Polymerisationsverfahren sind grundsätzlich kontinuierliche und diskontinuierliche Verfahren geeignet. Bevorzugt sind halbkontinuierliche Verfahren (Semi-batch-Verfahren), in denen nach Vermischen aller Komponenten Monomer oder Monomerengemische im Verlauf der Polymerisation nachdosiert werden.

Nach dem erfindungsgemäßen Verfahren werden zunächst wässrige Polymerdispersionen erhalten.

Die mittleren Teilchendurchmesser der Polymerpartikel in den erfindungsgemäßen Dispersionen betragen zwischen 10 und 1000 nm, bevorzugt zwischen 50 und 500 nm und besonders bevorzugt zwischen 70 und 350 nm. Die Verteilung der Teilchendurchmesser kann, muss aber nicht sehr einheitlich sein. Für manche Anwendungen, insbesondere für solche mit hohen Feststoffanteilen (> 55%), sind breite oder bimodale Verteilungen sogar bevorzugt.

Die nach dem erfindungsgemäßen Verfahren erhaltenen Polymere weisen technisch interessante Eigenschaften auf. Im Falle von Polyethylen weisen sie einen hohen Grad der Kristallinität auf, was beispielsweise durch die Anzahl der Verzweigungen nachgewiesen werden kann. Man findet weniger als 100 Verzweigungen, bevorzugt weniger als 50 Verzweigungen pro 1000 C-Atomen des Polymers, bestimmt durch ¹H-NMR und ¹³C-NMR-Spektroskopie.

Die Schmelzenthalpien der nach dem erfindungsgemäßen Verfahren erhältlichen Polyethylene sind größer 100 J/g, bevorzugt größer 140 und besonders bevorzugt größer als 180 J/g, gemessen durch DSC.

Die Molekulargewichtsverteilungen der nach dem erfindungsgemäßen Verfahren erhältlichen Polyethylene sind eng, d.h. die Q-Werte liegen zwischen 1,1 und 3,5 und bevorzugt zwischen 1,5 und 3,1.

Vorteilhaft an den erfindungsgemäßen Dispersionen ist neben dem günstigen Preis aufgrund der billigen Monomeren und Verfahren, dass sie witterungsstabiler als Dispersionen von Polybutadien oder Butadiencopolymeren sind. Gegenüber Dispersionen von Polymeren mit Acrylaten oder Methacrylaten als Hauptmonomer ist die geringere Neigung zum Verseifen als vorteilhaft zu nennen. Weiterhin ist von Vorteil, dass die meisten Olefine leichtflüchtig sind und sich nicht polymerisierte Restmonomere leicht entfernen lassen. Schließlich ist von Vorteil, dass während der Polymerisation keine Molmassenregler wie beispielsweise tert.-Dodecylmercaptan zugegeben werde müssen, die einerseits schlecht abgetrennt werden können und andererseits unangenehm riechen.

Aus den zunächst erhaltenen wässrigen Dispersionen lassen sich durch Entfernen des Wassers und gegebenenfalls des oder der organischen Lösemittel die Polymerpartikel als solche erhalten. Zur Entfernung des Wassers und gegebenenfalls des oder der organischen Lösemittel sind zahlreiche gängigen Verfahren geeignet, beispielsweise Filtrieren, Sprühtrocknen oder Verdampfen. Die so erhaltenen Polymere haben eine gute Morphologie und eine hohe Schüttdichte.

Die Teilchengröße lassen sich mit Lichtstreumethoden bestimmen. Einen Überblick findet man in D. Distler "Wäßrige Polymerdispersionen", Wiley-VCH Verlag, 1. Auflage, 1999, Kapitel 4.

Die erfindungsgemäßen Dispersionen lassen sich in zahlreichen Anwendungen vorteilhaft verwenden, wie beispielsweise Papieranwendungen wie Papierstreicherei oder Oberflächenleimung, weiterhin Anstrichen und Lacken, Bauchemikalien, Klebrohstoffe, Formschäume, Textil- und Lederapplikationen, Teppichrückenbeschichtungen, Matratzen oder pharmazeutischen Anwendungen.

Die folgenden Arbeitsbeispiele erläutern die Erfindung.

### Allgemeine Vorbemerkungen:

Alle Arbeiten wurden unter Ausschluß von Luft und Feuchtigkeit unter Verwendung von Standard-Schlenk-Techniken hergestellt. Geräte und Chemikalien waren entsprechend vorbereitet. Die Polymerviskosität wurde nach ISO 1628-3 bestimmt. Die Molmassen wurden mittels GPC bestimmt. Für die GPC-Untersuchungen wurden folgende Bedingungen in Anlehnung an DIN 55672 gewählt: Lösungsmittel-1,2,4-Trichlorbenzol, Fluss: 1 ml/min, Temperatur: 140 °C, Kalibrierung: PE-Standards, Gerät: Waters 150C. Die Anzahl der Methylgruppen wurde mittels IR-Spektroskopie bestimmt

Es werden folgende Abkürzungen verwendet: PE = Polyethylen, t-Bu: tert.-Butyl, Me = CH₃, Ph = C₆H₅, i-Pr = iso-Propyl, o-Tol = ortho-Toluyl,

### 1. Darstellung der erfindungsgemäßen Liganden:

### Allgemeine Arbeitsvorschrift:

Äquimolare Mengen der entsprechenden Salicylaldehyde und der Aminoazole wurden in möglichst wenig Methanol gelöst mit 0,5 ml Ameisensäure versetzt und anschließend 12 Stunden bei Raumtemperatur gerührt. In den meisten Fällen konnte das Produkt durch Abfiltrieren des entstandenen Niederschlages und mehrmaliges Waschen mit Methanol analysenrein isoliert werden. Im Falle der Verbindungen 1.1, 1.3, 1.4 und 1.5 wurde das Reaktionsgemisch am Rotationsverdampfer eingeengt und anschließend säulenchromatographisch gereinigt (Kieselgel Merck 60®, Laufmittel Hexan/ Ether 3:2).

Tabelle 1: Substitutionsmuster ausgewählter erfindungsgemäßer Liganden

**Tabelle 1**

| | | | | | | |
|---|---|---|---|---|---|---|
| 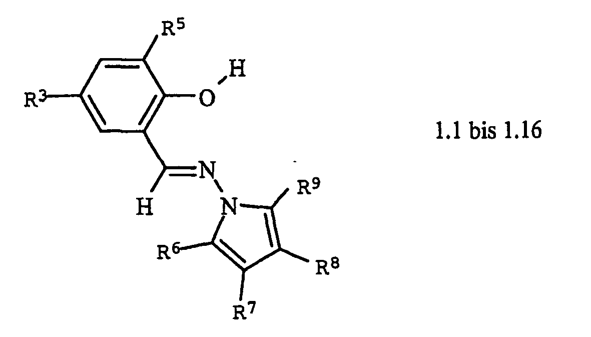 | | | | | | |

| Beispiel | R³ | R⁵ | R⁶ | R⁷ | R⁸ | R⁹ |
|---|---|---|---|---|---|---|
| 1.1 | H | H | Me | H | H | Me |
| 1.2 | H | H | Ph | H | H | Ph |
| 1.3 | H | H | Me | H | H | i-Pr |
| 1.4 | H | H | Me | H | H | Ph |
| 1.5 | H | H | Me | H | H | o-Tol |
| 1.6 | H | H | Ph | Ph | H Ph | |
| 1.7 | H | H | Me | H | -CH=CH-CH=CH- | |
| 1.8 | H | H | -CH=CH-CH=CH- | | -CH=CH-CH=CH- | |
| 1.10 | t-Bu | t-Bu | Ph | H | H | Ph |
| 1.11 | t-Bn | t-Bu | Me | H | H | i-Pr |
| 1.13 | t-Bu | t-Bu | Me | H | H | o-Tol |
| 1.14 | t-Bu | t-Bu | Ph | Ph | H | Ph |
| 1.15 | t-Bu | t-Bu | Me | H | -CH=CH-CH=CH- | |
| 1.16 | t-Bu | t-Bu | -CH=CH-CH=CH- | | -CH=CH-CH=CH- | |

Analytische Daten der Verbindungen 1.1 bis 1.16:
1.1: hell gelbes Pulver, Ausbeute 55% d. Theorie, Schmp. 43-45°C, C₁₃H₁₄N₂O. IR(KBr): 2938 w, 2898 w, 1622 s, 1609 m, 1588 w, 1528 m, 1458 w, 1451 w, 1398 s, 1358 m, 1327 w, 1285 s, 1259 s, 1184 w, 808 m, 781 m, 767 m, 746 s, 723 s, 555 m, 476 w cm⁻¹. ¹H NMR (CDCl₃, δ): 2.30 (6H, s, CH₃); 5.86 (2H, s, Pyrrol); 6.93-7.40 (4H, m, Phenyl); 8.45 (1H, s, Imin); 11.24 (1H, s, OH). ¹³C NMR (CDCl₃, δ): 13.0 (CH₃), 105.7, 117.1 (Pyrrol), 117.3, 119.6, 125.4, 132.0, 132.9 (Phenyl), 159.3 (Imin), 160.2 (Phenol).
1.2: hell gelbes Pulver, Ausbeute: 25% d. Theorie, Schmp. 122°C, C₂₃H₁₈N₂O. IR(KBr): 3058 w, 1622 s, 1601 s, 1568 m, 1487 s, 1458 m, 1451 m, 1387 w, 1317 w, 1304 m, 1273 s, 1225 w, 1194 m, 1151 w, 1034 w, 984 m, 918 s, 901 m, 820 m, 756 s, 737 s, 696 s, 480 m cm⁻¹. ¹H NMR (CDCl₃, δ): 6.42 (2H, s, Pyrrol); 6.70-7.51 (14H, m, Phenyl); 8.04 (1H, s, Imin); 10.83 (1H, s, OH). ¹³C NMR (CDCl₃, δ): 109.4, 116.7 (Pyrrol), 117.2, 119.5, 127.0, 128.4, 128.7, 132.0, 132.1, 132.3, 133.0 (Phenyl), 159.0 (Imin), 164.2 (Phenol).
1.3: hell gelbes Öl, Ausbeute: 36% d. Theorie, C₁₅H₁₈N₂O. MS(EI): m/z 242 (M⁺, 56%), 227 (M⁺-CH₃, 100%). IR(KBr): 2965 m, 2929 w, 1622 s, 1605 s, 1570 w, 1491 m, 1460 w, 1402 m, 1383 w, 1302 m, 1279 s, 1153 s, 1107 w, 1034 m, 997 w, 972 w, 906 m, 816 m, 754 s, 584 m, 480 m cm⁻¹. ¹H NMR (CDCl₃, δ): 1.26 (6H, d *J* = 6.78 Hz, CH(CH₃)₂); 2.33 (3H, s, CH₃); 3.06 (1H, septett *J* = 6.78 Hz, CH(CH₃)₂); 5.90 (1H, m *J* = 0.75 Hz, Pyrrol); 5.92 (1H, m *J* = 0.75 Hz, Pyrrol), 6.97-7.44 (4H, m, Phenyl); 8.50 (1H, s, Imin); 11.32 (1H, s, OH). ¹³C NMR (CDCl₃, δ): 13.3 (CH₃), 22.4 (CH(CH₃)₂), 25.7 (CH(CH₃)₂), 101.4, 106.4, 116.9 (Pyrrol), 117.3, 119.6, 123.6, 132.1, 133.1 (Phenyl), 137.9 (Pyrrol), 159.4 (Imin), 161.9 (Phenol).
1.4: hell gelbes Pulver, Ausbeute: 15% d. Theorie, Schmp. 66-69°C, C₁₈H₁₆N₂O. MS(EI): m/z 276 (M⁺, 58%), 156 (M⁺-HOC₆H₄CN, 100%). IR(KBr): 3060 w, 2923 w, 1622 s, 1605 s, 1570 w, 1516 m, 1491 m, 1474 m, 1445 w, 1395 m, 1292 s, 1271 s, 1188 w, 1153 m, 1074 w, 1034 m, 974 w, 903 w, 818 w, 750 s, 729 m, 698 s, 603 w, 509 w, 478 w cm⁻¹. ¹H NMR (CDCl₃, δ): 2.35 (3H, d *J* = 1.14 Hz, CH₃); 5.99 (1H, d x d *J =* 1.14 Hz, *J* = 3.78 Hz, Pyrrol); 6.22 (1H, d *J =* 3.78 Hz, Pyrrol), 6.78-7.48 (9H, m, Phenyl); 8.07 (1H, s, Imin); 11.13 (1H, s, OH). ¹³C NMR (CDCl₃, δ): 12.5 (CH₃), 106.1, 109.4, 116.8 (Pyrrol), 117.2, 119.5, 123.3, 126.5, 127.8, 128.7, 129.2, 132.0, 132.4, 132.9 (Phenyl, Pyrrol), 159.2 (Imin), 162.7 (Phenol).
1.5: hell gelbes Öl, Ausbeute: 95% d. Theorie, C₁₉H₁₈N₂O. MS(EI): m/z 290 (M⁺, 100%), 170 (M⁺-HOC₆H₄CN, 51%). IR(KBr): 3062 w, 2923 w, 1622 s, 1607 s, 1572 w, 1522 w, 1489 m, 1472 w, 1458 w, 1395 m, 1356 w, 1290 s, 1283 s, 1269 s, 1186 w, 1153 m, 1122 w, 1034 w, 958 w, 820 w, 754 s, 725 m, 607 w, 478 w cm⁻¹. ¹H NMR (CDCl₃, δ): 2.14 (3H, s, CH₃); 2.40 (3H, s, CH₃); 6.02 (1H, m *J* = 3.78 Hz, Pyrrol); 6.11 (1H, d *J* = 3.78 Hz, Pyrrol); 6.72-7.36 (8H, m, Phenyl); 7.86 (1H, s, Imin); 11.13 (1H, s, OH). ¹³C NMR (CDCl₃, δ): 12.5 (CH₃), 20.0 (CH₃), 105.3, 109.5 (Pyrrol), 117.0, 119.4, 126.2, 127.5, 128.1, 128.6, 130.5, 130.7, 131.7, 132.4, 132.6, 136.9 (Phenyl, Pyrrol), 157.7 (Imin), 158.8 (Phenol).
1.6: gelbes Pulver, Ausbeute: 72% d. Theorie, Schmp. 173-175°C; C₂₉H₂₂N₂O. MS(EI): m/z 414 (M⁺, 100%), 294 (M⁺-HOC₆H₄CN, 84%). IR(KBr): 3052 w, 1616 s, 1605 s, 1572 m, 1503 m, 1483 s, 1468 m, 1451 m, 1387 w, 1317 m, 1300 s, 1271 s, 1200 m, 1151 m, 1028 m, 964 m, 910 m, 837 w, 808 s, 764 s, 696 s, 673 m, 480 m cm⁻¹. ¹H NMR (CDCl₃, δ): 6.57 (1H, s, Pyrrol); 6.68-7.50 (19H, m, Phenyl); 7.91 (1H, s, Imin); 10.61 (1H, s, OH). ¹³C NMR (CDCl₃, δ): 110.1 (Pyrrol), 116.7, 117.1, 119.4, 122.7, 125.9, 127.2, 127.8, 128.1, 128.2, 128.3, 128.7, 128.8, 130.9, 131.0, 131.3, 131.8, 132.0, 132.9, 135.3, 136.9 (Phenyl, Pyrrol), 158.8 (Imin), 163.3 (Phenol).
1.7: hell gelbes Pulver, Ausbeute: 78% d. Theorie, Schmp. 150°C (Zersetzung), C₁₆H₁₄N₂O. MS (EI): m/z 250 (M⁺, 100%), 130 (M⁺-HOC₆H₄CN, 91%). IR(KBr): 3048 w, 1622 s, 1613 s, 1589 s, 1568 m, 1454 s, 1379 w, 1354 m, 1329 s, 1296 s, 1267 s, 1238 m, 1153 m, 1034 w, 787 m, 756 s, 740 s, 731 s, 712 m, 559 w, 544 m, 474 w cm⁻¹. ¹H NMR (CDCl₃, δ): (2 Rotamere im Verhältnis 0,8 : 1) 2.30 (3H, s, CH₃); 2.52 (3H, s, CH₃); 6.14 (1H, s, Indol); 6.35 (1H, s, Indol; 6.85-7.64 (16H, m, Phenyl); 8.96 (1H, s, Imin); 8.97 (1H, s, Imin); 11.12 (1H, s, OH); 11.20 (1H, s, OH). ¹³C NMR (CDCl₃, δ): 10.7, 12.9 (CH₃), 101.0, 109.5, 109.8, 111.0, 116.2, 117.3, 117.4, 117.5, 119.7, 120.6, 121.0, 121.2, 122.1, 122.3, 127.5, 127.8, 128.0, 128.3, 129.4, 131.6, 131.8, 132.2, 132.4, 132.7, 133.6, 136.0 (Phenyl, Indol), 155.8, 157.6 (Imin), 158.8, 158.9 (Phenol).
1.8: hell gelbes Pulver, Ausbeute: 68% d. Theorie, Schmp. 148°C, C₁₉H₁₄N₂O. IR(KBr): 3046 w, 1622 m, 1593 w, 1483 s, 1452 s, 1445 w, 1412 w, 1333 w, 1314 m, 1302 s, 1265 m, 1215 m, 1202 w, 1155 w, 941 m, 798 m, 740 s, 715 s, 696 m, 580 w, 542 m, 478 w cm⁻¹. ¹H NMR (CDCl₃, δ): 6.96-8.06 (12H, m, Phenyl); 9.03 (1H, s, Imin); 11.29 (1H, s, OH). ¹³C NMR (CDCl₃, δ): 109.9, 117.3, 117.7, 119.7, 120.4, 121.3, 122.6, 126.6, 131.4, 132.3, 137.6 (Phenyl), 154.7 (Imin), 158.7 (Phenol).
1.10: hell gelbes Pulver, Ausbeute: 63% d. Theorie, Schmp. 137°C, C₃₁H₃₄N₂O. MS(EI): m/z 450 (M⁺, 16%), 250 (100%), 219 (M⁺-Ph₂C₄H₂N, 34%), 130 (70%). IR(KBr): 3031 w, 2954 m, 2909 w, 1609 s, 1603 s, 1582 m, 1476 m, 1462 s, 1449 s, 1435 s, 1393 m, 1362 m, 1315 w, 1300 m, 1269 s, 1250 w, 1175 m, 972 w, 771 m, 758 s, 750 s, 700 s, 507 w cm⁻¹. ¹H NMR (CDCl₃, δ): 1.20 (9H, s, C(CH₃)₃); 1.44 (9H, s, C(CH₃)₃); 6.42 (2H, s, Pyrrol); 6.59 (1H, d *J* = 2.28 Hz, Phenyl); 7.23-7.54 (11H, m, Phenyl); 8.06 (1H, s, Imin); 11.34 (1H, s, OH). ¹³C NMR (CDCl₃, δ): 29.4, 31.3 (C(CH₃)₃), 34.0, 35.1 (C(CH₃)₃), 108.9, 115.9 (Pyrrol), 126.7, 126.8, 128.2, 128.3, 128.6, 132.1, 136.9, 141.0 (Phenyl), 156.5 (Imin), 166.7 (Phenol).
1.11: hell gelbes Pulver, Ausbeute: 85% d. Theorie, Schmp. 134°C, C₂₃H₃₄N₂O. MS(EI): m/z 354 (M⁺, 75%), 339 (M⁺-CH₃, 100%). IR(KBr): 3006 w, 2961 m, 2942 m, 2871 w, 1611 s, 1588 w, 1520 w, 1472 s, 1456 m, 1439 s, 1398 s, 1383 m, 1362 w, 1308 m, 1273 s, 1259 m, 1250 s, 1232 w, 1173 s, 1001 w, 758 s, 729 s, 511 w cm⁻¹. ¹H NMR (CDCl₃, δ): 1.23 (6H, d *J* = 6.78 Hz, CH(CH₃)₂); 1.33 (9H, s, C(CH₃)₃); 1.48 (9H, s, C(CH₃)₃); 2.31 (3H, s, CH₃); 3.04 (1H, Septett *J* = 6.78 Hz, CH(CH₃)₂); 5.86 (1H, m, Pyrrol); 5.90 (1H, m, Pyrrol); 7.12 (1H, d *J* = 2.25 Hz, Phenyl); 7.50 (1H, d *J* = 2.25 Hz, Phenyl); 8.49 (1H, s, Imin); 11.67 (1H, s, OH). ¹³C NMR (CDCl₃, δ): 13.1 (CH₃), 22.5 (CH(CH₃)₂), 25.7 (CH(CH₃)₂), 29.4, 31.4 (C(CH₃)₃), 34.2, 35.2 (C(CH₃)₃), 101.0, 105.7, 116.1 (Pyrrol), 123.7, 126.6, 128.3, 137.3, 137.7, 141.2 (Phenyl, Pyrrol), 156.7 (Imin), 164.5 (Phenol).
1.13: gelbes Pulver, Ausbeute: 88% d. Theorie, Schmp. 164°C, C₂₇H₃₄N₂O. MS(EI): m/z 402 (M⁺, 44%), 362 (100%), 130 (32%). IR(KBr): 3015 w, 2963 s, 2909 w, 1613 m, 1586 w, 1470 m, 1435 s, 1395 w, 1360 s, 1288 s, 1281 s, 1250 m, 1175 w, 958 w, 876 w, 816 w, 764 s, 758 s, 723 m, 712 w, 646 m, 623 w, 453 w cm⁻¹. ¹H NMR (CDCl₃, δ): 1.21 (9H, s, C(CH₃)₃); 1.45 (9H, s, C(CH₃)₃); 2.15 (3H, s, CH₃); 2.40 (3H, s, CH₃); 6.01 (1H, m *J* = 3.78 Hz, Pyrrol); 6.11 (1H, m *J* = 3.78 Hz, Pyrrol); 6.51 (1H, m *J* = 2.75 Hz, Phenyl); 7.16-7.37 (5H, m, Phenyl); 7.86 (1H, s, Imin); 11.44 (1H, s, OH). ¹³C NMR (CDCl₃, δ): 12.5, 20.1 (CH₃), 29.4, 31.3 (C(CH₃)₃), 34.0, 35.1 (C(CH₃)₃), 105.5, 109.0, 116.2 (Pyrrol), 126.1, 126.3, 127.5, 127.8, 128.4, 130.5, 130.8, 132.7, 136.7, 137.0, 140.9 (Phenyl, Pyrrol), 156.0 (Imin), 159.9 (Phenol).
1.14: hell gelbes Pulver, Ausbeute: 90% d. Theorie, Schmp. 164°C, C₄₃H₃₉N₂O. IR(KBr): 3058 w, 2957 m, 2907 w, 1609 s, 1582 m, 1481 s, 1466 m, 1452 m, 1437 s, 1391 w, 1362 w, 1317 w, 1302 m, 1273 m, 1252 s, 1202 w, 1177 m, 1028 w, 802 m, 766 s, 702 s, 684 w, 501 w cm⁻¹. ¹H NMR (CDCl₃, δ) : 1.18 (9H, s, C(CH₃)₃); 1.40 (9H, s, C(CH₃)₃); 6.51 (1H, m *J* = 2.25 Hz, Phenyl); 6.62 (1H, s, Pyrrol); 7.10-7.56 (16H, m, Phenyl); 7.97 (1H, s, Imin); 11.13 (1H, s, OH). ¹³C NMR (CDCl₃, δ): 29.3, 31.3 (C(CH₃)₃), 34.0, 35.1 (C(CH₃)₃), 109.5 (Pyrrol), 115.9, 122.4, 125.8, 126.6, 127.0, 127.5, 128.0, 128.1, 128.2, 128.3, 128.6, 128.7, 130.8, 131.1, 131.4, 131.9, 135.6, 136.8, 140.9 (Phenyl, Pyrrol), 156.3 (Imin), 165.7 (Phenol).
1.15: hell gelbes Pulver, Ausbeute: 83% d. Theorie, Schmp. 126°C, C₂₄H₃₀N₂O. MS (EI) : m/z 362 (M⁺, 100%), 347 (M⁺-CH₃, 18%), 130 (33%). IR(KBr): 3050 w, 2954 s, 2907 w, 2869 w, 1609 m, 1589 w, 1476 s, 1447 s, 1393 w, 1381 w, 1362 m, 1333 m, 1298 s, 1267 w, 1250 s, 1236 s, 1178 m, 1132 m, 766 s, 737 s, 725 s, 708 m, 528 w cm⁻¹. ¹H NMR (CDCl₃, δ): 1.37 (9H, s, C(CH₃)₃); 1.51 (9H, s, C(CH₃)₃); 2.54 (3H, s, CH₃); 6.33 (1H, Indol); 7.12-7.63 (6H, m, Phenyl); 8.97 (1H, s, Imin); 11.58 (1H, s, OH). ¹³C NMR (CDCl₃, δ): 12.92 (CH₃), 29.4, 31.5 (C(CH₃)₃), 34.2, 35.2 (C(CH₃)₃), 100.4 109.6, 116.6, 120.5, 120.9, 121.8, 126.3, 127.6, 132.4, 135.9, 137.1, 141.3 (Phenyl, Indol), 156.1 (Imin), 158.7 (Phenol).
1.16: hell gelbes Pulver, Ausbeute: 66% d. Theorie, Schmp. 200°C, C₂₇H₃₀N₂O. MS(EI): m/z 398 (M⁺, 60%), 383 (M⁺-CH₃, 15%), 167 (M⁺-HO(t-Bu)₂C₆H₂CN, 100%). IR(KBr): 3062 w, 2952 m, 2907 w, 2869 w, 1624 w, 1603 m, 1483 s, 1449 s, 1389 w, 1362 m, 1321 w, 1302 s, 1250 w, 1215 m, 1204 w, 1177 m, 920 m, 806 m, 767 m, 739 s, 713 s, 698 s, 684 w, 644 w, 418 w cm⁻¹. ¹H NMR (CDCl₃, δ): 1.38 (9H, s, C(CH₃)₃); 1.54 (9H, s, C(CH₃)₃); 7.20-8.09 (10H, m, Phenyl); 9.12 (1H, s, Imin); 11.72 (1H, s, OH). ¹³C NMR (CDCl₃, δ): 29.5, 31.5 (C(CH₃)₃), 34.3, 35.2 (C(CH₃)₃), 109.8, 116.7, 120.4, 121.0, 122.4, 126.2, 126.5, 127.5, 137.2, 137.7, 141.4 (Phenyl, Indol), 156.0 (Imin), 157.7 (Phenol).

### 2. Synthese der Beta(pyrrolimino)enolat-Nickel-Komplexe:

### Allgemeine Arbeitsvorschrift:

Die Beta(pyrrolimino)enole 1.1. bis 1.16 wurden in THF gelöst und mit einer äquimolaren Menge n-Butyllithium bei -80°C deprotoniert. Nach Erwärmen auf Raumtemperatur wurde das Lösungsmittel abgezogen und der Rückstand in Benzol gelöst. Anschließend wurde die Reaktionslösung mit einem Äquivalent Ni(PPh₃)₂PhCl versetzt und 12 Stunden bei Raumtemperatur gerührt. Das entstandene Lithiumchlorid wurde mittels Filtration durch Celite® abgetrennt, anschließend wurde die Reaktionslösung eingeengt und das Produkt durch Zugabe von Petrolether ausgefällt. Mehrmaliges Waschen mit Petrolether und Methanol ergab ein analysenreines Produkt.

**Tabelle 2: Substitutionsmuster ausgewählter erfindungsgemäßer Komplexe**

| | | | | | | |
|---|---|---|---|---|---|---|
| 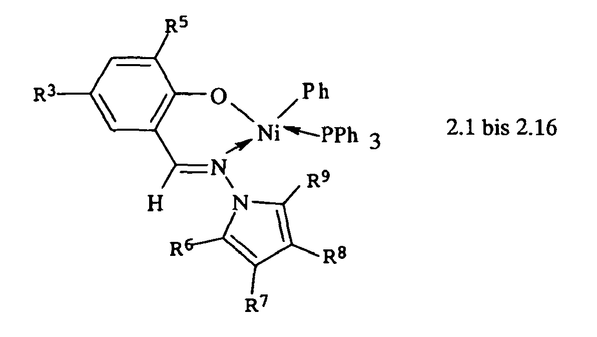 | | | | | | |

| Beispiel | R³ | R⁵ | R⁶ R⁷ | | R⁸ R⁹ | |
|---|---|---|---|---|---|---|
| 2.1 | H | H | Me | H | H | Me |
| 2.2 | H | H | Ph | H | H | Ph |
| 2.3 | H | H | Me | H | H | i-Pr |
| 2.4 | H | H | Me | H | H | Ph |
| 2.5 | H | H | Me | H | H | o-Tol |
| 2.6 | H | H | Ph | Ph | H Ph | |
| 2.7 | H | H | Me | H | -CH=CH-CH=CH- | |
| 2.8 | H | H | -CH=CH-CH=CH- | | -CH=CH-CH=CH- | |
| 2.10 | t-Bu | t-Bu | Ph | H | H | Ph |
| 2.11 | t-Bu | t-Bu | Me | H | H | i-Pr |
| 2.13 | t-Bu | t-Bu | Me | H | H | o-Tol |
| 2.14 | t-Bu | t-Bu | Ph | Ph | H | Ph |
| 2.15 | t-Bu | t-Bu | Me | H | -CH=CH-CH=CH- | |
| 2.16 | t-Bu | t-Bu | -CH=CH-CH=CH- | | -CH=CH-CH=CH- | |

Analytische Daten der erfindungsgemäßen Komplexe 2.1 bis 2.16
2.1: gelbes Pulver, Ausbeute: 54% d. Theorie, Schmp. 158°C, C₃₇H₃₃N₂NiOP. IR(KHr): 3050 w, 2913 w, 1611 s, 1578 s, 1562 m, 1528 s, 1466 m 1441 s, 1368 w, 1341 w, 1146 m, 1128 w, 1097 m, 1020 w, 931 m, 742 s, 727 s, 704 s, 694 s, 551 m, 532s, 509 m, 495 m cm⁻¹. ¹H NMR (C₆D₆, δ): 2.32 (6H, s, CH₃), 5.60 (2H, s, Pyrrol); 6.32-7.77 (25H, m, Phenyl, Imin). ¹³C NMR (C₆D₆, δ): 12.6 (CH₃), 103.1 (Pyrrol), 114.7, 117.4, 122.3, 123.3, 124.4, 125.2, 128.1, 128.8, 129.8, 131.2, 131.8, 132.3, 132.5, 134.2, 134.6, 134.7, 135.4, 137.4, 145.4, 146.1, 167.7, 170.2 (Phenyl, Pyrrol, Imin). ³¹P NMR (C₆D₆, δ): 26.3.
2.2: orangefarbenes Pulver, Ausbeute: 78% d. Theorie, Schmp. 275-280°C (Zersetzung), C₄₇H₃₇N₂NiOP. IR(KBr): 3054 w, 1611 s, 1574 m, 1564 w, 1526 m, 1481 w, 1468 m, 1437 s, 1371 w, 1342 w, 1186 w, 1146 w, 1111 w, 1097 m, 756 s, 740 s, 729 s, 694 s, 542 w, 530 m, 509 s, 499 s cm⁻¹. ¹H NMR (C₆D₆, δ): 6.23 (2H, s, Pyrrol) ; 6.27-8.00 (35H, m, Phenyl). ¹³C NMR (C₆D₆, δ): Aufgrund paramagnetischer Effekte und der geringen Löslichkeit der Verbindung war keine Auswertung möglich. ³¹P NMR (C₆D₆, δ): 26.4.
2.3: gelbes Pulver, Ausbeute: 58% d. Theorie, Schmp. 162°C, C₃₉H₃₇N₂NiOP. IR(KBr): 3054 w, 2959 w, 2923 w, 1611 s, 1578 s, 1562 m, 1530 m, 1466 m, 1443 m, 1368 w, 1342 m, 1223 m, 1204 w, 1146 m, 1095 m, 1020 m, 935 m, 750 s, 727 s, 692 s, 569 w, 530 s, 509 m, 495 m cm⁻¹. ¹H NMR (C₆D₆, δ): 1.36 (3H, d *J* = 6.67 Hz, CH(CH₃)₂); 1.57 (3H, d *J* = 6.67 Hz, CH(CH₃)₂); 2.57 (3H, s, CH₃); 3.86 (1H, Septett *J* = 6.67 Hz, CH(CH₃)₂); 5.83 (1H, m, Pyrrol); 5.90 (1H, m, Pyrrol), 6.50-8.19 (25H, m, Phenyl, Imin). ¹³C NMR (C₆D₆, δ): 12.0 (CH₃), 21.1, 25.1, 25.7 (CH(CH₃)₂), 99.0, 102.6 (Pyrrol), 114.2, 116.6, 121.6, 122.6, 123.4, 124.8, 129.2, 130.5, 131.1, 133.5, 134.0, 134.1, 134.8, 135.4, 136.8, 167.0, 170.1 (Phenyl, Pyrrol, Imin). ³¹P NMR (C₆D₆, δ): 26.0.
2.4: orangefarbenes Pulver, Ausbeute: 55% d. Theorie, Schmp. 145°C, C₄₂H₃₅N₂NiOP. IR(KBr): 3054 w, 1609 s, 1572 s, 1562 m, 1526 m, 1514 s, 1464 m, 1439 m, 1341 w, 1204 w, 1194 w, 1180 w, 1146 m, 1121 m, 1097 m, 758 m, 739 s, 727 s, 692 s, 542 m, 532 m, 509 w cm⁻¹. ¹H NMR (C₆D₆, δ): 2.27 (3H, s, CH₃) ; 5.76 (2H, s, Pyrrol); 6.25-8.50 (30H, m, Phenyl, Imin). ¹³C NMR (C₆D₆, δ): 12.7 (CH₃), 104.7, 106.0 (Pyrrol), 114.8, 117.3, 122.2, 123.4. 125.6, 128.8, 129.7, 129.8, 130.6, 131.1, 131.7, 132.3, 132.4, 132.5, 133.3, 134.0, 134.2, 134.3, 134.6, 134.7, 135.6, 136.9, 138.4, 145.9, 167.9, 170.5 (Phenyl, Pyrrol, Imin). ³¹P NMR (C₆D₆, δ): 26.4.
2.5: orangefarbenes Pulver, Ausbeute: 13% d. Theorie, Schmp. 175°C, C₄₃H₃₇N₂NiOP. IR(KBr): 3054 w, 1597 s, 1574 w, 1562 w, 1528 m, 1483 s, 1468 m, 1437 s, 1371 w, 1348 w, 1186 s, 1151 m, 1121 s, 1095 w, 852 w, 764 s, 719 s, 694 s, 586 w, 540 s cm⁻¹. ¹H NMR (C₆D₆, δ): 1.97 (3H, s, CH₃); 2.41 (3H, s, CH₃); 5.66 (1H, m *J* = 3.75 Hz, Pyrrol); 6.08 (1H, m *J* = 4.20 Hz, Pyrrol); 6.28-8.33 (29H, m, Phenyl, Imin). ¹³C NMR (C₆D₆, δ): 12.4 (CH₃), 22.1 (CH₃), 103.8, 108.4 (Pyrrol), 114.7, 117.4, 122.3, 123.5, 125.8. 126.4, 126.8, 128.1, 128.6, 129.7 (d J_{PC} = 11.55 Hz), 131.1, 131.3, 131.5, 131.7, 132.9, 134.0, 134.1, 134.3, 134.5, 134.7, 135.5, 137.4, 144.9, 145.5, 167.7, 170.3 (Phenyl, Pyrrol, Imin). ³¹P NMR (C₆D₆, δ): 25.0.
2.6: orangefarbenes Pulver, Schmp. 167°C, Ausbeute: 47% d. Theorie, C₅₃H₄₁N₂NiOP. IR(KBr): 3052 w, 1611 s, 1572 m, 1528 w, 1503 w, 1483 w, 1466 m, 1437 s, 1342 w, 1261 w, 1204 w, 1148 m, 1095 m, 1028 m, 931 w, 802 w, 754 s, 731 m, 694 s, 530 m, 511 w cm⁻¹. ¹H NMR (C₆D₆, δ): 6.03 (1H, m, Pyrrol); 6.10-7.55 (40H, m, Phenyl, Imin). ¹³C NMR (C₆D₆, δ): 107.7(Pyrrol), 114.9, 116.6, 122.3, 123.5, 125.1, 125.8, 126.3, 127.0, 127.9, 128.5, 128.7, 128.8, 129.0, 129.3, 129.6, 129.7, 130.9, 131.5, 131.6, 132.0, 132.5, 133.2, 134.0, 134.1, 134.3, 134.5, 134.6, 135.7, 137.6, 137.9, 138.5, 138.8, 144.1, 144.8, 167.9, 171.8 (Phenyl, Pyrrol, Imin). ³¹P NMR (C₆D₆, δ): 25.8.
2.7: gelbes Pulver, Ausbeute: 54% d. Theorie, Schmp. 155°C, C₄₀H₃₃N₂NiOP. IR(KBr): 3052 w, 1609 s, 1564 m, 1526 m, 1481 w, 1466 m, 1454 w, 1437 s, 1337 w, 1308 w, 1148 w, 1148 w, 1121 m, 1095 m, 1020 w, 928 w, 739 s, 694 s, 542 s, 530 s, 509 m, 495 w cm⁻¹. ¹H NMR (C₆D₆, δ): 2.02 (3H, s, CH₃); 5.50-7.50 (30H, m, Phenyl, Imin). ¹³C NMR (C₆D₆, δ): 12.1 (CH₃), 109.6 (Pyrrol), 114.8, 119.8, 129.8, 131.7, 132.3, 134.3, 134.3, 134.5, 134.7, weitere Signale aufgrund paramagnetischer Effekte nicht beobachtet (Phenyl, Pyrrol, Imin). ³¹P NMR (C₆D₆, δ): 26.4.
2.8: orangefarbenes Pulver, Ausbeute: 48% d. Theorie, Schmp. 149°C, C₄₃H₃₃N₂NiOP. IR(KBr): 3052 w, 1609 m, 1591 w, 1483 m, 1466 w, 1439 s, 1314 w, 1190 s, 1165 m, 1121 s, 1095 w, 1072 w, 1026 w, 997 m, 843 w, 752 s, 721 s, 696 s, 544 s, 499 m, 451 w cm⁻¹. ¹H NMR (C₆D₆, δ): 7.01-7.83 (33H, m, Phenyl, Imin). ¹³C NMR (C₆D₆, δ): 125.8, 126.8, 128.4, 128.6, 128.8, 131.5, 131.6, 132.3, 132.4, 133.6, 134.0, 134.3, 134.9, 146.1, 147.4, 159.9 (Carbazol, Phenyl, Imin). ³¹P NMR (C₆D₆, δ): 25.9.
2.10: orangefarbenes Pulver, Ausbeute: 56% d. Theorie, Schmp. 170°C, C₅₅H₅₃N₂NiOP. IR(KBr): 3052 w, 2956 w, 1615 m, 1599 m, 1574 s, 1545 w, 1524 s, 1483 m, 1458 w, 1435 s, 1416 s, 1256 m, 1196 s, 1167 m, 1121 s, 1095 w, 758 w, 744 s, 729 s, 694 s, 546 s, 530 m, 511 w cm⁻¹. ¹H NMR (C₆D₆, δ): 0.97 (9H, s, C(CH₃)₃); 1.20 (9H, s, C(CH₃)₃); 6.30 (2H, s, Pyrrol); 6.40-8.35 (33H, m, Phenyl, Imin). ¹³C NMR (C₆D₆, δ): 29.8, 31.2 (C(CH₃)₃), 33.8, 35.0 (C(CH₃)₃), 107.2 (Pyrrol), 117.0, 121.8, 125.2, 126.3, 127.9, 128.5, 128.6, 128.7, 129.8, 131.6, 131.7, 132.3, 132.4, 132.6, 133.5, 134.8, 135.0, 135.1, 136.0, 136.9, 137.0, 142.0, 166.6, 171.7 (Phenyl, Pyrrol, Imin). ³¹P NMR (C₆D₆, δ): 25.1.
2.11: orangefarbenes Pulver, Ausbeute: 52% d. Theorie, Schmp. 158°C, C₄₇H₅₃N₂NiOP. IR(KBr): 3056 w, 2959 s, 2907 w, 2869 w, 1616 m, 1578 s, 1564 w, 1526 w, 1460 m, 1435 s, 1420 s, 1360 m, 1329 w, 1271 w, 1256 m, 1169 w, 1097 m, 744 m, 729 s, 532 s, 511 m, 473 w cm⁻¹. ¹H NMR (C₆D₆, δ): 1.13 (9H, s, C(CH₃)₃); 1.36 (3H, d *J* = 6.78 Hz, CH(CH₃)₂); 1.46 (9H, s, C(CH₃)₃); 1.58 (3H, d *J* = 6.67 Hz, CH(CH₃)₂); 2.49 (3H, s, CH₃); 4.03 (1H, Septett *J* = 6.78 Hz, CH(CH₃)₂); 5.87 (2H, m, Pyrrol); 6.57-8.19 (23H, m, Phenyl, Imin). ¹³C NMR (C₆D₆, δ): 12.4 (CH₃), 22.2, 25.6, 26.5 (CH(CH₃)₂), 29.9, 31.4 (C(CH₃)₃), 33.9, 35.0 (C(CH₃)₃), 99.7, 103.1 (Pyrrol), 117.3, 121.9, 124.2, 128.1, 130.0, 131.1, 131.7, 132.3, 135.0, 135,2, 136.0, 136.3, 141.9, 145.0, 145.7, 166.4, 171.1 (Phenyl, Pyrrol, Imin). ³¹P NMR (C₆D₆, δ): 24.4.
2.13: orangefarbenes Pulver, Ausbeute: 43% d. Theorie, Schmp. 176°C, C₅₁H₅₃N₂NiOP. IR(KBr): 3056 w, 2961 m, 2903 w, 1616 m, 1578 s, 1526 s, 1435 s, 1422 s, 1259 s, 1169 w, 1097 s, 1020 s, 877 w, 802 s, 744 m, 727 m, 702 s, 692 s, 532 s, 513 m, 493 m cm⁻¹. ¹H NMR (C₆D₆, δ): 0.89 (9H, s, C(CH₃)₃); 1.24 (9H, s, C(CH₃)₃); 1.77 (3H, s, CH₃); 2.45 (3H, s, CH₃); 5.69 (1H, m *J* = 3.75 Hz, Pyrrol); 6.15 (1H, m *J* = 3.75 Hz, Pyrrol); 6.53-8.00 (27H, m, Phenyl, Imin). ¹³C NMR (C₆D₆, δ): 11.8 (CH₃), 21.9 (CH₃), 29.9, 31.4 (C(CH₃)₃), 33.9, 34.9 (C(CH₃)₃), 103.8, 108.1 (Pyrrol), 117.6, 122.0, 125.9, 126.4, 126.8, 129.3, 129.8, 131.1, 131.2, 131.3, 131.4, 131.9, 132.1, 133.4, 134.9, 135.1, 135.7, 137.6, 142.4, 144.8, 145.5, 166.1, 170.2 (Phenyl, Pyrrol, Imin). ³¹P NMR (C₆D₆, δ): 24.0.
2.14: orangefarbenes Pulver, Ausbeute: 62% d. Theorie, Schmp. 206°C, C₆₁H₅₇N₂NiOP. IR(KBr): 3052 w, 2954 m, 2903 w, 1615 w, 1601 w, 1574 m, 1526 m, 1483 w, 1437 s, 1420 m, 1360 w, 1331 w, 1258 m, 1175 w, 1095 w, 764 m, 754 m, 729 s, 694 s, 542 m, 532 s, 511 m, 493 w cm⁻¹. ¹H NMR (C₆D₆, δ): 0.83 (9H, s, C(CH₃)₃); 1.19 (9H, s, C(CH₃)₃); 6.34 (1H, m, Pyrrol), 6.50-8.26 (38H, m, Phenyl, Imin). ¹³C NMR (C₆D₆, δ): 29.8, 31.3 (C(CH₃)₃), 33.9, 34.9 (C(CH₃)₃), 107.6 (Pyrrol), 116.6, 121.9, 122.1, 124.9, 125.7, 126.4, 127.1, 127.8, 128.5, 128.8, 129.6, 129.8, 131.5, 131.8, 132.0, 132.4, 132.6, 133.1, 134.9, 135.1, 135.9, 136.8, 137.1, 137.6, 142.1, 166.5, 171.8 (Phenyl, Pyrrol, Imin). ³¹P NMR (C₆D₆, δ): 24.3.
2.15: gelbes Pulver, Ausbeute: 57% d. Theorie, Schmp. 166°C, C₄₈H₄₉N₂NiOP. IR(KBr): 3052 w, 2957 m, 2869 w, 1615 m, 1578 s, 1551 w, 1528 s, 1478 w, 1458 m, 1435 s, 1422 s, 1356 w, 1331 w, 1258 m, 1175 w, 1097 m, 1020 w, 742 m, 729 s, 692 s, 530 s, 511 m, 493 w cm⁻¹. ¹H NMR (C₆D₆, δ): 0.92 (9H, s, C(CH₃)₃); 1.27 (9H, s, C(CH₃)₃); 2.29 (3H, s, CH₃); 5.85 (1H, m, Indol); 6.21-7.87 (27H, m, Phenyl, Imin). ¹³C NMR (C₆D₆, δ): 12.6 (CH₃), 29.9, 31.5 (C(CH₃)₃), 33.9, 35.0 (C(CH₃)₃), 97.4, 109.6 (Pyrrol), 117.6, 119.7, 120.9, 121.9, 125.9, 127.8, 128.1, 128.6, 129.9, 130.0, 131.3, 131.8, 132.5, 134.1, 135.0, 135.2, 136.0, 136.4, 142.1, 145.3, 146.0, 166.8, 171.8 (Phenyl, Pyrrol, Imin). ³¹P NMR (C₆D₆, δ): 26.2.
2.16: orangefarbenes Pulver, Ausbeute: 73% d. Theorie, Schmp. 165°C, C₅₁H₄₉N₂NiOP. IR(KBr): 3052 w, 2956 m, 2905 w, 1616 m, 1574 s, 1528 s, 1483 w, 1449 s, 1435 s, 1422 s, 1331 w, 1317 w, 1258 w, 1234 m, 1173 m, 1095 m, 845 w, 742 s, 729 s, 719 w, 692 s, 530 s, 493 m cm⁻¹. ¹H NMR (C₆D₆, δ): 0.95 (9H, s, C(CH₃)₃); 1.28 (9H, s, C(CH₃)₃); 5.63-7.99 (31H, m, Phenyl, Imin). ¹³C NMR (C₆D₆, δ): 29.9, 31.5 (C(CH₃)₃), 34.0, 35.0 (C(CH₃)₃), 109.4, 117.9, 119.1, 119.9, 120.8, 121.7, 124.2, 125.5, 128.7, 128.8, 129.9, 131.3, 132.0, 132.5, 135.0, 135.2, 136.1, 136.6, 139.9, 142.2, 143.7, 144.5, 145.9, 167.1, 172.9 (Carbazol, Phenyl, Imin). ³¹P NMR (C₆D₆, δ): 26.6.

Standardverfahren für die Polymerisation mit den Komplexen 2.1 bis 2.16:

In einen 1-1-Stahlautoklaven wurden 400 ml Toluol vorgelegt. Dazu wurden äquimolare Mengen des Nickelkomplexes und Ni(COD)₂ gegeben. Dieses Reaktionsgemisch wurde für 30 min bei 30°C gerührt, dann wurde Ethylen bis zu einem Druck von 4 MPa aufgepresst. Nach 90 min wurde die Reaktion durch Entspannen abgestoppt und das Polymerpulver isoliert.

Einzelheiten zu den Polymerisationen sind Tabelle 3 zu entnehmen.

**Tabelle 3 (Polymerisationen mit Nickelkomplexen):**

| Komplexverbin-dung | Ansatz (mmol) | Ausbeute PE (g) | eta-Wert (dl/g) | Mw | Mn | Mw/Mn | Summe |
|---|---|---|---|---|---|---|---|
| | | | | (g/mol) | (g/mol) | | CH₃ |
| 2.2 | 0,086 | 8,3 | 2,27 | 275437 | 12742 | 21,6 | 29 |
| 2.6 | 0,095 | 3,0 | 0,38 | n.b. | n.b. | n.b. | 40,6 |
| 2.7 | 0,096 | 0,7 | 1,5 | 102933 | 3902 | 26,4 | 34,5 |
| 2.10 | 0,096 | 12,0 | 0,57 | 14407 | 8012 | 1,8 | 39,7 |
| 2.11 | 0,092 | 25,0 | 0,97 | 33220 | 10884 | 3,0 | 26,1 |
| 2.14 | 0,089 | 12,3 | 0,38 | 12744 | 6873 | 1,85 | 35 |
| 2.15 | 0,087 | 25,0 | 0,1 | n.b. | n.b. | n.b. | 82 |
| V1 | 0,093 | 0,5 | 0,47 | n.b. | n.b. | n.b. | 23,7 |

| | | | | | | | |
|---|---|---|---|---|---|---|---|
| n.b.: nicht bestimmt, V1: Ni(Me)(PPh₃)-{η²-1-[C(H)=*N*(2,6-di-i-Pr-Ph)]-2-*O*-C₆H₄ (hergestellt in Analogie zu WO 98/42664) | | | | | | | |

## Patentansprüche

1. Komplexverbindungen der allgemeinen Formel I, 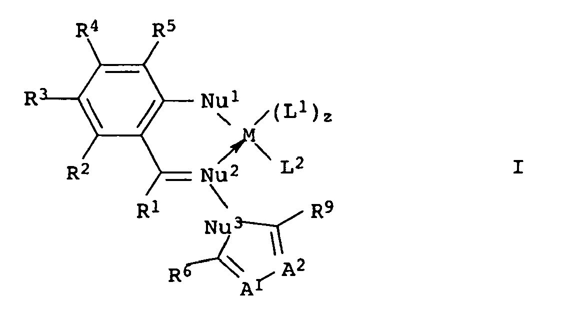 bei denen die Variablen wir folgt definiert sind:
M ein Element der 6. bis 10. Gruppe des Periodensystems der Elemente, ausser Nickel
Nu¹ ausgewählt aus O, S oder Se;
Nu², Nu³ ausgewählt aus N oder P,
A¹ N oder C-R⁷ oder Si-R⁷,
A² N oder C-R⁸ oder Si-R⁸,
R¹ bis R⁹ ausgewählt aus
Wasserstoff,
C₁-C₈-Alkyl, substituiert oder unsubstituiert,
C₂-C₈-Alkenyl, substituiert oder unsubstituiert, mit ein bis 4 isolierten oder konjugierten Doppelbindungen;
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy, substituiert oder unsubstituiert,
C₆-C₁₄-Aryloxy,
SiR¹⁰R¹¹R¹² oder O-SiR¹⁰R¹¹R¹²;
fünf- bis sechsgliedrigen stickstoffhaltigen Heteroarylresten, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy,
C₆-C₁₄-Aryloxy,
SiR¹⁰R¹¹R¹² oder O-SiR¹⁰R¹¹R¹²;
wobei räumlich benachbarte Reste R¹ bis R⁹ miteinander zu einem 5- bis 12-gliedrigen Ring verbunden sein können;
L¹ ein organischer oder anorganischer Neutralligand,
L² ein organischer oder anorganischer anionischer Ligand, wobei L¹ und L² miteinander durch eine oder mehrere kovalente Bindungen verknüpft sein können,
z eine ganze Zahl von 1 bis 3,
R¹⁰ bis R¹² gleich oder verschieden und ausgewählt werden aus Wasserstoff, C₁-C₈-Alkyl, C₃-C₁₂-Cycloalkyl, C₇-C₁₃-Aralkyl oder C₆-C₁₄-Aryl.

2. Komplexverbindungen der allgemeinen Formel I, 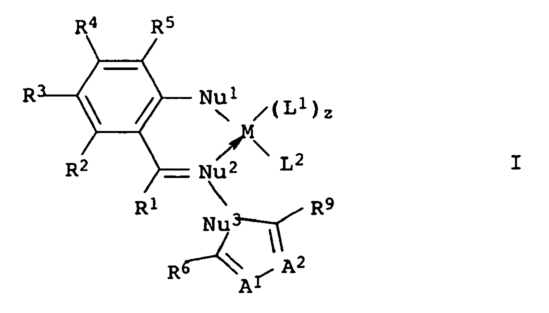 bei denen die Variablen wir folgt definiert sind:
M Nickel
Nu¹ ausgewählt aus O, S oder Se;
Nu², Nu³ ausgewählt aus N oder P,
A¹ N oder Si-R⁷,
A² N oder C-R⁸ oder Si-R⁸,
R¹ bis R⁹ ausgewählt aus
Wasserstoff,
C₁-C₈-Alkyl, substituiert oder unsubstituiert,
C₂-C₈-Alkenyl, substituiert oder unsubstituiert, mit ein bis 4 isolierten oder konjugierten Doppelbindungen;
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy, substituiert oder unsubstituiert,
C₆-C₁₄-Aryloxy,
SiR¹⁰R¹¹R¹² oder O-SiR¹⁰R¹¹R¹²;
fünf- bis sechsgliedrigen stickstoffhaltigen Heteroarylresten, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy,
C₆-C₁₄-Aryloxy,
SiR¹⁰R¹¹R¹² oder O-SiR¹⁰R¹¹R¹²;
wobei räumlich benachbarte Reste R¹ bis R⁹ miteinander zu einem 5- bis 12-gliedrigen Ring verbunden sein können;
L¹ ein organischer oder anorganischer Neutralligand,
L² ein organischer oder anorganischer anionischer Ligand, wobei L¹ und L² miteinander durch eine oder mehrere kovalente Bindungen verknüpft sein können,
z eine ganze Zahl von 1 bis 3,
R¹⁰ bis R¹² gleich oder verschieden und ausgewählt werden aus Wasserstoff, C₁-C₈-Alkyl, C₃-C₁₂-Cycloalkyl, C₇-C₁₃-Aralkyl oder C₆-C₁₄-Aryl.

3. Komplexverbindungen nach Anspruch 1 und 2, wobei
L¹ ausgewählt wird aus
Phosphanen (R¹³)ₓPH₃₋ₓ ,
Aminen (R¹³)ₓNH₃₋ₓ,
Ethern (R¹³)₂O,
H₂O,
Alkoholen (R¹³)OH,
Pyridin,
Pyridinderivaten der Formel C₅H₅₋ₓ(R¹³)ₓN,
CO,
C₁-C₁₂-Alkylnitrilen,
C₆-C₁₄-Arylnitrilen oder
ethylenisch ungesättigten Doppelbindungssystemen,
wobei x eine ganze Zahl von 0 bis 3 bedeutet;
L² ausgewählt wird aus
Halogenidionen,
Amidionen (R¹³)ₓ₋₁NH₂₋ₓ,
C₁-C₆-Alkylanionen,
Allylanionen,
Benzylanionen oder Arylanionen;
die Reste R¹³ gleich oder verschieden sind und ausgewählt werden aus Wasserstoff, C₁-C₈-Alkyl, C₃-C₁₂-Cycloalkyl, C₇-C₁₃-Aralkyl oder C₆-C₁₄-Aryl.

4. Komplexverbindungen nach den Ansprüchen 1 bis 3, bei denen die Variablen wie folgt definiert sind:
R¹, R², R⁴ sind Wasserstoff,
R³, R⁵ sind unabhängig voneinander Wasserstoff oder C₁-C₈-Alkyl, verzweigt oder unverzweigt,
R⁶, R⁹ C₁-C₈-Alkyl, gleich oder verschieden, verzweigt oder unverzweigt.

5. Komplexverbindungen mit folgender Formel, 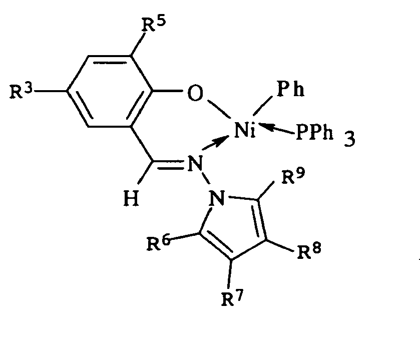 wobei die Variablen wie folgt definiert sind:
R³ und R⁵ sind Wasserstoff und R⁷ und R⁸ sind Wasserstoff und
R⁶ und R⁹ sind gleich und Methyl oder Phenyl oder R⁶ ist Methyl und R⁹ ist iso-Propyl, Phenyl oder ortho-Tolyl oder R³, R⁵ und R⁸ sind Wasserstoff und R⁶, R⁷ und R⁹ sind Phenyl oder
R³, R⁵ und R⁷ sind Wasserstoff und R⁶ ist Methyl und R⁸ und R⁹ sind zusammen eine Gruppe -CH=CH-CH=CH- oder
R³ und R⁵ sind Wasserstoff und R⁶ und R⁷ sind zusammen eine Gruppe -CH=CH-CH=CH- und R⁸ und R⁹ sind zusammen eine Gruppe -CH=CH-CH=CH- oder
R³ und R⁵ sind tert. Butyl und R⁷ und R⁸ sind Wasserstoff und R⁶ und R⁹ sind Phenyl oder R⁶ ist Methyl und R⁹ ist iso-Propyl oder ortho-Tolyl oder
R³ und R⁵ sind tert. Butyl und R⁸ ist Wasserstoff und R⁶, R⁷ und R⁹ sind Phenyl oder
R³ und R⁵ sind tert. Butyl und R⁷ ist Wasserstoff und R⁶ ist Methyl und R⁸ und R⁹ sind zusammen eine Gruppe -CH=CH-CH=CH- oder
R³ und R⁵ sind tert. Butyl und R⁶ und R⁷ sind zusammen eine Gruppe -CH=CH-CH=CH- und R⁸ und R⁹ sind zusammen eine Gruppe -CH=CH-CH=CH-.

6. Liganden der allgemeinen Formel, 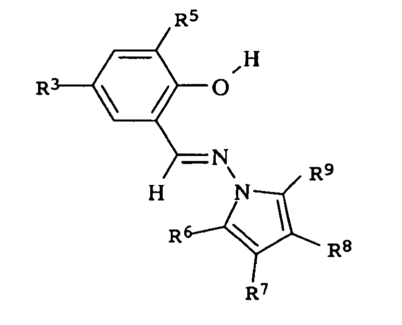 wobei die Variablen wie folgt definiert sind:
R³ und R⁵ sind Wasserstoff und R⁷ und R⁸ sind Wasserstoff und
R⁶ und R⁹ sind gleich und Methyl oder Phenyl oder R⁶ ist Methyl und R⁹ ist iso-Propyl, Phenyl oder ortho-Tolyl oder R³, R⁵ und R⁸ sind Wasserstoff und R⁶, R⁷ und R⁹ sind Phenyl oder
R³, R⁵ und R⁷ sind Wasserstoff und R⁶ ist Methyl und R⁸ und R⁹ sind zusammen eine Gruppe -CH=CH-CH=CH- oder
R³ und R⁵ sind Wasserstoff und R⁶ und R⁷ sind zusammen eine Gruppe -CH=CH-CH=CH- und R⁸ und R⁹ sind zusammen eine Gruppe -CH=CH-CH=CH- oder
R³ und R⁵ sind tert. Butyl und R⁷ und R⁸ sind Wasserstoff und R⁶ und R⁹ sind Phenyl oder R⁶ ist Methyl und R⁹ ist iso-Propyl oder ortho-Tolyl oder
R³ und R⁵ sind tert. Butyl und R⁸ ist Wasserstoff und R⁶, R⁷ und R⁹ sind Phenyl oder
R³ und R⁵ sind tert. Butyl und R⁷ ist Wasserstoff und R⁶ ist Methyl und R⁸ und R⁹ sind zusammen eine Gruppe -CH=CH-CH=CH- oder
R³ und R⁵ sind tert. Butyl und R⁶ und R⁷ sind zusammen eine Gruppe -CH=CH-CH=CH- und R⁸ und R⁹ sind zusammen eine Gruppe -CH=CH-CH=CH-.

7. Verfahren zur Herstellung von Komplexverbindungen der allgemeinen Formel I 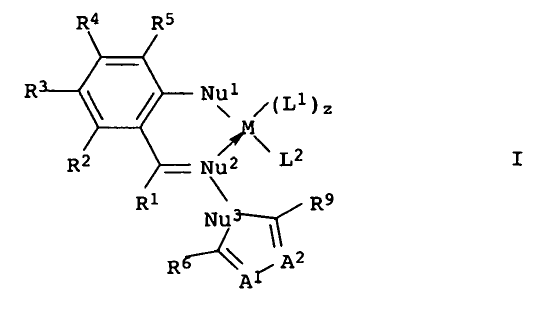 bei denen die Variablen wir folgt definiert sind:
M ein Element der 6. bis 10. Gruppe des Periodensystems der Elemente,
Nu¹ ausgewählt aus O, S oder Se;
Nu², Nu³ ausgewählt aus N oder P,
A¹ N oder C-R⁷ oder Si-R⁷,
A² N oder C-R⁸ oder Si-R⁸,
R¹ bis R⁹ ausgewählt aus
Wasserstoff,
C₁-C₈-Alkyl, substituiert oder unsubstituiert,
C₂-C₈-Alkenyl, substituiert oder unsubstituiert, mit ein bis 4 isolierten oder konjugierten Doppelbindungen;
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy, substituiert oder unsubstituiert,
C₆-C₁₄-Aryloxy,
SiR¹⁰R¹¹R¹² oder O-SiR¹⁰R¹¹R¹²;
fünf- bis sechsgliedrigen stickstoffhaltigen Heteroarylresten, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy,
C₆-C₁₄-Aryloxy,
SiR¹⁰R¹¹R¹² oder O-SiR¹⁰R¹¹R¹²;
wobei räumlich benachbarte Reste R¹ bis R⁹ miteinander zu einem 5- bis 12-gliedrigen Ring verbunden sein können;
L¹ ein organischer oder anorganischer Neutralligand,
L² ein organischer oder anorganischer anionischer Ligand,
wobei L¹ und L² miteinander durch eine oder mehrere kovalente Bindungen verknüpft sein können,
z eine ganze Zahl von 1 bis 3, R¹⁰ bis R¹² gleich oder verschieden und ausgewählt werden aus Wasserstoff, C₁-C₈-Alkyl, C₃-C₁₂-Cycloalkyl, C₇-C₁₃-Aralkyl oder C₆-C₁₄-Aryl,
**dadurch gekennzeichnet, dass** man einen Liganden der allgemeinen Formel II 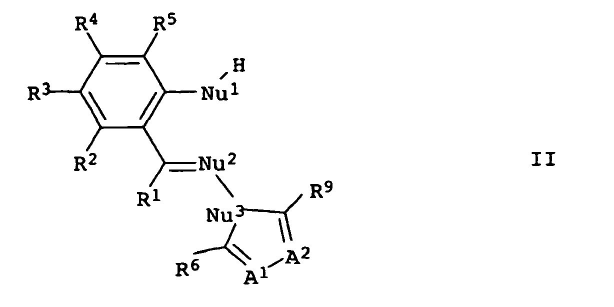 zunächst mit Hilfe einer Base deprotoniert und anschließend mit 0,2 bis 5 Äquivalenten einer Metallverbindung MX₄, MX₃, ML¹L² oder MX₂ umsetzt, wobei X Halogen, C₁-C₈-Alkyl, C₃-C₁₂-Cycloalkyl, C₇-C₁₃-Aralkyl oder C₆-C₁₄-Aryl bedeutet und wobei MX₂, MX₃ oder MX₄ durch weitere Neutralliganden stabilisiert sind.

8. Verfahren zur Herstellung von Komplexverbindungen der allgemeinen Formel I gemäß Anspruch 7, **dadurch gekennzeichnet dass** M ein Element der 6. bis 10. Gruppe des Periodensystems der Elemente, ausser Nickel ist und dass MX₂, MX₃ oder MX₄ optional durch weitere Neutralliganden stabilisiert sind.

9. Verfahren zur Herstellung von Komplexverbindungen der allgemeinen Formel I gemäß Anspruch 7, **dadurch gekennzeichnet, dass** als Base Methyllithium, Ethyllithium, n-Butyllithium, sec-Butyllithium, tert.-Butyllithium oder Hexyllithium, Grignard-Verbindungen, Lithiumamid, Kaliumamid, Kaliumhydrid oder Lithiumdiisopropylamid verwendet wird und MX₂, MX₃ oder MX₄ optional durch weitere Neutralliganden stabilisiert sind.

10. Verfahren zur Polymerisation oder Copolymerisation von Olefinen unter Verwendung von Komplexverbindungen gemäß den Ansprüchen 1 bis 4.

11. Verfahren zur Herstellung eines Trägerkatalysators für die Polymerisation oder Copolymerisation von Olefinen, **dadurch gekennzeichnet, dass** man eine oder mehrere Komplexverbindungen nach den Ansprüchen 1 bis 4 und optional einen Aktivator auf einem festen Träger abscheidet.

12. Trägerkatalysator für die Polymerisation oder Copolymerisation von Olefinen gemäß Anspruch 11.

13. Verfahren zur Polymerisation oder Copolymerisation von Olefinen unter Verwendung eines Trägerkatalysators gemäß Anspruch 11.

14. Verfahren zur Emulsionspolymerisation oder Emulsionscopolymerisation von Ethylen oder anderen 1-Olefinen und optional weiteren Olefinen unter Verwendung einer Komplexverbindung der allgemeinen Formel I, wobei M ein Element der 6. bis 10. Gruppe des Periodensystems der Elemente ist.

## Claims

1. A complex of the formula I, 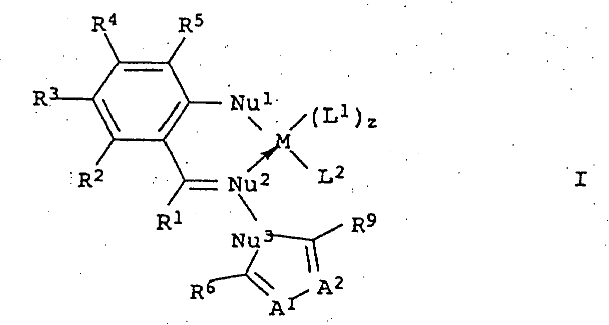 where the variables are defined as follows:
M is an element of groups 6 to 10 of the Periodic Table of the Elements, apart from nickel,
Nu¹ is selected from among O, S and Se;
Nu², Nu³ are selected from among N and P,
A¹ is N or C-R⁷ or Si-R⁷,
A² is N or C-R⁸ or Si-R⁸,
R¹ to R⁹ are selected from among
hydrogen,
C₁-C₈-alkyl, substituted or unsubstituted,
C₂-C₈-alkenyl, substituted or unsubstituted and having from one to 4 isolated or conjugated double bonds;
C₃-C₁₂-cycloalkyl, substituted or unsubstituted,
C₇-C₁₃-aralkyl,
C₆-C₁₄-aryl, unsubstituted or monosubstituted or polysubstituted by identical or different substituents selected from among
C₁-C₈-alkyl, substituted or unsubstituted,
C₃-C₁₂-cycloalkyl,
C₇-C₁₃-aralkyl,
C₆-C₁₄-aryl,
halogen,
C₁-C₆-alkoxy, substituted or unsubstituted,
C₆-C₁₄-aryloxy,
SiR¹⁰R¹¹R¹² and O-SiR¹⁰R¹¹R¹²;
five- and six-membered nitrogen-containing heteroaryl radicals, unsubstituted or monosubstituted or polysubstituted by identical or different substituents selected from among
C₁-C₈-alkyl, substituted or unsubstituted,
C₃-C₁₂-cycloalkyl,
C₇-C₁₃-aralkyl,
C₆-C₁₄-aryl,
halogen,
C₁-C₆-alkoxy,
C₆-C₁₄-aryloxy,
SiR¹⁰R¹¹R¹² and O-SiR¹⁰R¹¹R¹²;
where adjacent radicals R¹ to R⁹ may be joined to one another to form a 5- to 12-membered ring;
L¹ is an uncharged organic or inorganic ligand,
L² is an organic or inorganic anionic ligand,
where L¹ and L² may be joined to one another by one or more covalent bonds,
z is an integer from 1 to 3,
R¹⁰ to R¹² are identical or different and are selected from among hydrogen, C₁-C₈-alkyl, C₃-C₁₂-cycloalkyl, C₇-C₁₃-aralkyl and C₆-C₁₄-aryl.

2. A complex of the formula I, 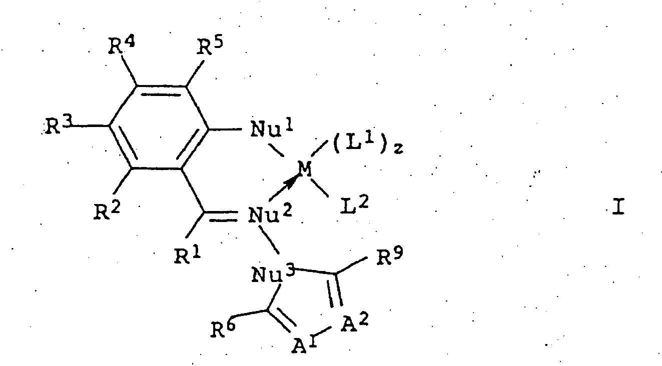 where the variables are defined as follows:
M is nickel,
Nu¹ is selected from among O, S and Se;
Nu², Nu³ are selected from among N and P,
A¹ is N or Si-R⁷,
A² is N or C-R⁸ or Si-R⁸,
R¹ to R⁹ are selected from among
hydrogen,
C₁-C₈-alkyl, substituted or unsubstituted,
C₂-C₈-alkenyl, substituted or unsubstituted and having from one to 4 isolated or conjugated double bonds;
C₃-C₁₂-cycloalkyl, substituted or unsubstituted,
C₇-C₁₃-aralkyl,
C₆-C₁₄-aryl, unsubstituted or monosubstituted or polysubstituted by identical or different substituents selected from among
C₁-C₈-alkyl, substituted or unsubstituted,
C₃-C₁₂-cycloalkyl,
C₇-C₁₃-aralkyl,
C₆-C₁₄-aryl,
halogen,
C₁-C₆-alkoxy, substituted or unsubstituted,
C₆-C₁₄-aryloxy,
SiR¹⁰R¹¹R¹² and O-SiR¹⁰R¹¹R¹²;
five- and six-membered nitrogen-containing heteroaryl radicals, unsubstituted or monosubstituted or polysubstituted by identical or different substituents selected from among
C₁-C₈-alkyl, substituted or unsubstituted,
C₃-C₁₂-cycloalkyl,
C₇-C₁₃-aralkyl,
C₆-C₁₄-aryl,
halogen,
C₁-C₆-alkoxy,
C₆-C₁₄-aryloxy,
SiR¹⁰R¹¹R¹² and O-SiR¹⁰R¹¹R¹²;
where adjacent radicals R¹ to R⁹ may be joined to one another to form a 5- to 12-membered ring;
L¹ is an uncharged organic or inorganic ligand,
L² is an organic or inorganic anionic ligand, where L¹ and L² may be joined to one another by one or more covalent bonds,
z is an integer from 1 to 3,
R¹⁰ to R¹² are identical or different and are selected from among hydrogen, C₁-C₈-alkyl, C₃-C₁₂-cycloalkyl, C₇-C₁₃-aralkyl and C₆-C₁₄-aryl.

3. The complex according to claim 1 or 2, wherein
L¹ is selected from among
phosphines (R¹³)ₓPH₃₋ₓ,
amines (R¹³)ₓNH₃₋ₓ,
ethers (R¹³)₂O,
H₂O,
alcohols (R¹³)OH,
pyridine,
pyridine derivatives of the formula C₅H₅₋ₓ(R¹³)ₓN,
CO,
C₁-C₁₂-alkyl nitriles,
C₆-C₁₄-aryl nitriles and
ethylenically unsaturated double bond systems,
where x is an integer from 0 to 3;
L² is selected from among
halide ions,
amide ions (R¹³)ₓ₋₁NH₂₋ₓ,
C₁-C₆-alkyl anions,
allyl anions,
benzyl anions and aryl anions;
the radicals R¹³ are identical or different and are selected from among hydrogen, C₁-C₈-alkyl, C₃-C₁₂-cycloalkyl, C₇-C₁₃-aralkyl and C₆-C₁₄-aryl.

4. The complex according to any of claims 1 to 3 in which the variables are defined as follows:
R¹, R² , R⁴ are each hydrogen,
R³, R⁵ are each, independently of one another, hydrogen or C₁-C₈-alkyl, branched or unbranched,
R⁶, R⁹ are each C₁-C₈-alkyl, identical or different, branched or unbranched.

5. A complex having the following formula, 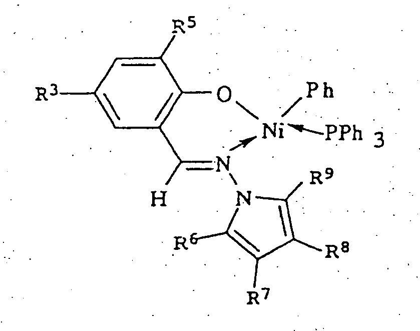 where the variables are defined as follows:
R³ and R⁵ are each hydrogen and R⁷ and R⁸ are each hydrogen and R⁶ and R⁹ are identical and are each methyl or phenyl or R⁶ is methyl and R⁹ is isopropyl, phenyl or ortho-tolyl or
R³, R⁵ and R⁸ are each hydrogen and R⁶, R⁷ and R⁹ are each phenyl or
R³, R⁵ and R⁷ are each hydrogen and R⁶ is methyl and R⁸ and R⁹ are together a -CH=CH-CH=CH- group or
R³ and R⁵ are each hydrogen and R⁶ and R⁷ are together a -CH=CH-CH=CH- group and R⁸ and R⁹ are together a -CH=CH-CH=CH- group or
R³ and R⁵ are each tert-butyl and R⁷ and R⁸ are each hydrogen and R⁶ and R⁹ are each phenyl or R⁶ is methyl and R⁹ is isopropyl or ortho-tolyl or
R³ and R⁵ are each tert-butyl and R⁸ is hydrogen and R⁶, R⁷ and R⁹ are each phenyl or
R³ and R⁵ are each tert-butyl and R⁷ is hydrogen and R⁶ is methyl and R⁸ and R⁹ are together a -CH=CH-CH=CH- group or
R³ and R⁵ are each tert-butyl and R⁶ and R⁷ are together a -CH=CH-CH=CH- group and R⁸ and R⁹ are together a -CH=CH-CH=CH- group.

6. A ligand of the general formula, 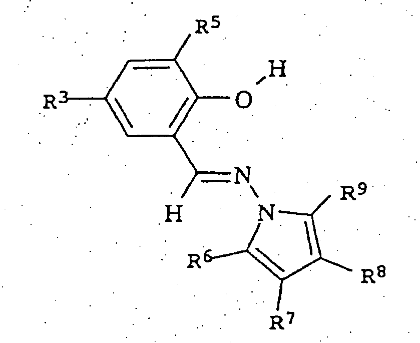 where the variables are defined as follows:
R³ and R⁵ are each hydrogen and R⁷ and R⁸ are each hydrogen and R⁶ and R⁹ are identical and are each methyl or phenyl or R⁶ is methyl and R⁹ is isopropyl, phenyl or ortho-tolyl or
R³, R⁵ and R⁸ are each hydrogen and R⁶, R⁷ and R⁹ are each phenyl or
R³, R⁵ and R⁷ are each hydrogen and R⁶ is methyl and R⁸ and R⁹ are together a -CH=CH-CH=CH- group or
R³ and R⁵ are each hydrogen and R⁶ and R⁷ are together a -CH=CH-CH=CH- group and R⁸ and R⁹ are together a -CH=CH-CH=CH- group or
R³ and R⁵ are each tert-butyl and R⁷ and R⁸ are each hydrogen and R⁶ and R⁹ are each phenyl or R⁶ is methyl and R⁹ is isopropyl or ortho-tolyl or
R³ and R⁵ are each tert-butyl and R⁸ is hydrogen and R⁶, R⁷ and R⁹ are each phenyl or
R³ and R⁵ are each tert-butyl and R⁷ is hydrogen and R⁶ is methyl and R⁸ and R⁹ are together a -CH=CH-CH=CH- group or
R³ and R⁵ are each tert-butyl and R⁶ and R⁷ are together a -CH=CH-CH=CH- group and R⁸ and R⁹ are together a -CH=CH-CH=CH- group.

7. A process for preparing a complex of the formula I, 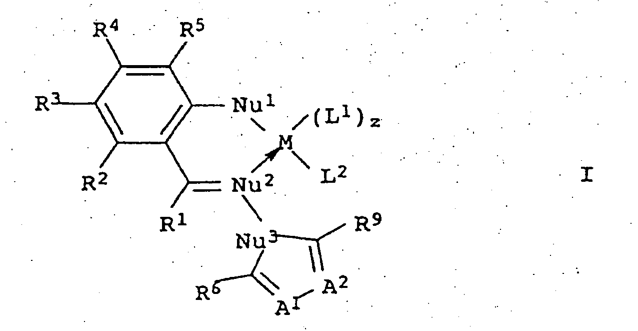 where the variables are defined as follows:
M is an element of groups 6 to 10 of the Periodic Table of the Elements,
Nu¹ is selected from among O, S and Se;
Nu², Nu³ are selected from among N and P,
A¹ is N or C-R⁷ or Si-R⁷,
A² is N or C-R⁸ or Si-R⁸,
R¹ to R⁹ are selected from among
hydrogen,
C₁-C₈-alkyl, substituted or unsubstituted,
C₂-C₈-alkenyl, substituted or unsubstituted and having from one to 4 isolated or conjugated double bonds;
C₃-C₁₂-cycloalkyl, substituted or unsubstituted,
C₇-C₁₃-aralkyl,
C₆-C₁₄-aryl, unsubstituted or monosubstituted or polysubstituted by identical or different substituents selected from among
C₁-C₈-alkyl, substituted or unsubstituted,
C₃-C₁₂-cycloalkyl,
C₇-C₁₃-aralkyl,
C₆-C₁₄-aryl,
halogen,
C₁-C₆-alkoxy, substituted or unsubstituted,
C₆-C₁₄-aryloxy,
SiR¹⁰R¹¹R¹² and O-SiR¹⁰R¹¹R¹²;
five- and six-membered nitrogen-containing heteroaryl radicals, unsubstituted or monosubstituted or polysubstituted by identical or different substituents selected from among
C₁-C₈-alkyl, substituted or unsubstituted,
C₃-C₁₂-cycloalkyl,
C₇-C₁₃-aralkyl,
C₆-C₁₄-aryl,
halogen,
C₁-C₆-alkoxy,
C₆-C₁₄-aryloxy,
SiR¹⁰R¹¹R¹² and O-SiR¹⁰R¹¹R¹²;
where adjacent radicals R¹ to R⁹ may be joined to one another to form a 5- to 12-membered ring;
L¹ is an uncharged organic or inorganic ligand,
L² is an organic or inorganic anionic ligand, where L¹ and L² may be joined to one another by one or more covalent bonds,
z is an integer from 1 to 3,
R¹⁰ to R¹² are identical or different and are selected from among hydrogen, C₁-C₈-alkyl, C₃-C₁₂-cycloalkyl, C₇-C₁₃-aralkyl and C₆-C₁₄-aryl,
which comprises firstly deprotonating a ligand of the formula II 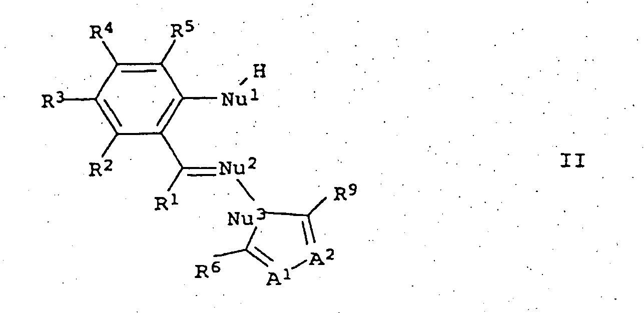 by means of a base and subsequently reacting the product with from 0.2 to 5 equivalents of a metal compound MX₄, MX₃, ML¹L² or MX₂, where X is halogen, C₁-C₈-alkyl, C₃-C₁₂-cycloalkyl, C₇-C₁₃-aralkyl or C₆-C₁₄-aryl and MX₂, MX₃ or MX₄ are stabilized by further uncharged ligands.

8. The process for preparing a complex of the formula I according to claim 7, wherein M is an element of groups 6 to 10 of the Periodic Table of the Elements, apart from nickel, and MX₂, MX₃ or MX₄ are optionally stabilized by further uncharged ligands.

9. The process for preparing a complex of the formula I according to claim 7, wherein methyllithium, ethyllithium, n-butyllithium, sec-butyllithium, tert-butyllithium or hexyllithium, Grignard compounds, lithium amide, potassium amide, potassium hydride or lithium diisopropylamide is used as base and MX₂, MX₃ or MX₄ are optionally stabilized by further uncharged ligands.

10. A process for the polymerization or copolymerization of olefins using complexes according to any of claims 1 to 4.

11. A process for preparing a supported catalyst for the polymerization or copolymerization of olefins, which comprises depositing one or more complexes according to any of claims 1 to 4 and, if desired, an activator on a solid support.

12. A supported catalyst for the polymerization or copolymerization of olefins as set forth in claim 11.

13. A process for the polymerization or copolymerization of olefins using a supported catalyst according to claim 11.

14. A process for the emulsion polymerization or emulsion copolymerization of ethylene or other 1-olefins and, if desired, further olefins using a complex of the formula I, where M is an element of groups 6 to 10 of the Periodic Table of the Elements.

## Revendications

1. Composés complexes de formule générale I 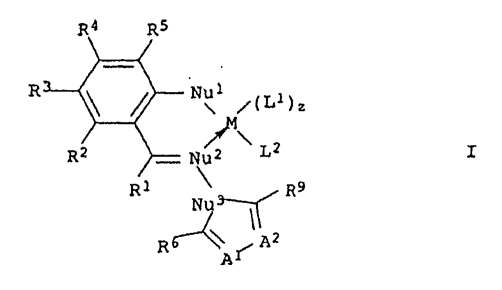 dans lesquels les variables sont définies comme suit :
M est un élément du 6ème au 10ème Groupe du Tableau Périodique des Eléments, à l'exception du nickel,
Nu¹ est choisi parmi O, S ou Se ;
Nu², Nu³ sont choisis parmi N ou P,
A¹ est N ou C-R⁷ ou Si-R⁷,
A² est N ou C-R⁸ ou Si-R⁸,
R¹ à R⁹ sont choisis parmi l'atome d'hydrogène ; les groupes alkyle en C₁-C₈, substitués ou non substitués ; les groupes alcényle en C₂-C₈, substitués ou non substitués, ayant 1 à 4 doubles liaisons isolées
ou conjuguées ; les groupes cycloalkyle en C₃-C₁₂, substitués ou non substitués ; les groupes aralkyle en C₇-C₁₃ ; les groupes aryle en C₆-C₁₄, non substitués, ou une ou plusieurs fois substitués, par des substituants identiques ou différents choisis parmi les groupes alkyle en C₁-C₈ substitués ou non substitués, les groupes cycloalkyle en C₃-C₁₂, les groupes aralkyle en C₇-C₁₃, les groupes aryle en C₆-C₁₄, les atomes d'halogène, les groupes alcoxy en C₁-C₆ substitués ou non substitués, les groupes aryloxy en C₆-C₁₄, SiR¹⁰R¹¹R¹² ou O-SiR¹⁰R¹¹R¹² ;
les radicaux hétéroaryle azotés à cinq à six chaînons, non substitués, ou une ou plusieurs fois substitués par des substituants identiques ou différents choisis parmi les groupes alkyle en C₁-C₈ substitués ou non substitués, les groupes cycloalkyle en C₃-C₁₂, les groupes aralkyle en C₇-C₁₃, les groupes aryle en C₆-C₁₄, les atomes d'halogène, les groupes alcoxy en C₁-C₆, les groupes aryloxy en C₆-C₁₄, SiR¹⁰R¹¹R¹² ou O-SiR¹⁰R¹¹R¹² ;
où les radicaux spatialement voisins R¹ à R⁹ peuvent être reliés les uns aux autres pour donner un noyau à 5 à 12 chaînons ;
L¹ est un ligand neutre organique ou inorganique,
L² est un ligand anionique organique ou inorganique, L¹ et L² pouvant être reliés l'un à l'autre par une ou plusieurs liaisons covalentes,
z est un nombre entier de 1 à 3,
R¹⁰ à R¹² sont identiques ou différents et sont choisis parmi l'atome d'hydrogène, les groupes alkyle en C₁-C₈, cycloalkyle en C₃-C₁₂, aralkyle en C₇-C₁₃ ou aryle en C₆-C₁₄.

2. Composés complexes de formule générale I 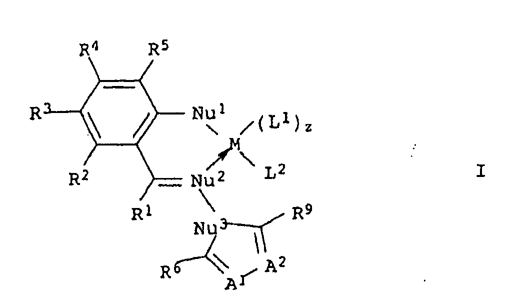 dans lesquels les variables sont définies comme suit :
M est le nickel,
Nu¹ est choisi parmi O, S ou Se,
Nu², Nu³ sont choisis parmi N ou P,
A¹ est N ou Si-R⁷,
A² est N ou C-R⁸ ou Si-R⁸,
R¹ à R⁹ sont choisis parmi l'atome d'hydrogène ; les groupes alkyle en C₁-C₈, substitués ou non substitués ; les groupes alcényle en C₂-C₈, substitués ou non substitués, ayant 1 à 4 doubles liaisons isolées ou conjuguées ; les groupes cycloalkyle en C₃-C₁₂, substitués ou non substitués ; les groupes aralkyle en C₇-C₁₃ ; les groupes aryle en C₆-C₁₄, non substitués, ou une ou plusieurs fois substitués, par des substituants identiques ou différents choisis parmi les groupes alkyle en C₁-C₈ substitués ou non substitués, les groupes cycloalkyle en C₃-C₁₂, les groupes aralkyle en C₇-C₁₃, les groupes aryle en C₆-C₁₄, les atomes d'halogène, les groupes alcoxy en C₁-C₆ substitués ou non substitués, les groupes aryloxy en C₆-C₁₄, SiR¹⁰R¹¹R¹² ou O-SiR¹⁰R¹¹R¹² ;
les radicaux hétéroaryle azotés à cinq à six chaînons, non substitués, ou une ou plusieurs fois substitués par des substituants identiques ou différents choisis parmi les groupes alkyle en C₁-C₈ substitués ou non substitués, les groupes cycloalkyle en C₃-C₁₂, les groupes aralkyle en C₇-C₁₃, les groupes aryle en C₆-C₁₄, les atomes d'halogène, les groupes alcoxy en C₁-C₆, les groupes aryloxy en C₆-C₁₄, SiR¹⁰R¹¹R¹² ou O-SiR¹⁰R¹¹R¹² ;
où les radicaux spatialement voisins R¹ à R⁹ peuvent être reliés les uns aux autres pour donner un noyau à 5 à 12 chaînons ;
L¹ est un ligand neutre organique ou inorganique,
L² est un ligand anionique organique ou inorganique, L¹ et L² pouvant être reliés l'un à l'autre par une ou plusieurs liaisons covalentes,
z est un nombre entier de 1 à 3,
R¹⁰ à R¹² sont identiques ou différents et sont choisis parmi l'atome d'hydrogène, les groupes alkyle en C₁-C₈, cycloalkyle en C₃-C₁₂, aralkyle en C₇-C₁₃ ou aryle en C₆-C₁₄.

3. Composés complexes selon les revendications 1 et 2, dans lesquels :
L¹ est choisi parmi les phosphanes (R¹³)ₓPH₃₋ₓ, les amines (R¹³)ₓNH₃₋ₓ, les éthers (R¹³)₂O, H₂O, les alcools (R¹³)OH, la pyridine, les dérivés de la pyridine de formule C₅H₅₋ₓ(R¹³)ₓN, CO, les (alkyle en C₁₋C₁₂)nitriles, les (aryle en C₆-C₁₄)nitriles, ou les systèmes de doubles liaisons à insaturation éthylénique, x étant un nombre entier de 0 à 3 ;
L² est choisi parmi les ions halogénure, les ions amide (R¹³)ₓ₋₁NH₂₋ₓ, les anions alkyle en C₁-C₆, les anions allyle, les anions benzyle ou les anions aryle ;
les radicaux R¹³ étant identiques ou différents et étant choisis parmi l'atome d'hydrogène et les groupes alkyle en C₁-C₈, cycloalkyle en C₃-C₁₂, aralkyle en C₇-C₁₃ ou aryle en C₆-C₁₄.

4. Composés complexes selon les revendications 1 à 3, dans lesquels les variables sont définies comme suit :
R¹, R² et R⁴ sont des atomes d'hydrogène,
R³ et R⁵ représentent chacun indépendamment de l'autre un atome d'hydrogène ou un groupe alkyle en C₁₋C₈ à chaîne droite ou ramifiée,
R⁶ et R⁹ sont des groupes alkyle en C₁-C₈, identiques ou différents, à chaîne droite ou ramifiée.

5. Composés complexes ayant la formule suivante : 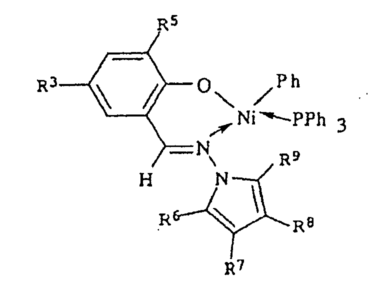 dans laquelle les variables sont définies comme suit :
R³ et R⁵ sont des atomes d'hydrogène, et R⁷ et R⁸ sont des atomes d'hydrogène, et R⁶ et R⁹ sont identiques et représentent des groupes méthyle ou phényle, ou bien R⁶ est le groupe méthyle et R⁹ est le groupe isopropyle, phényle ou orthotolyle, ou bien
R³, R⁵ et R⁸ sont des atomes d'hydrogène, et R⁶, R⁷ et R⁹ sont des groupes phényle, ou bien
R³, R⁵ et R⁷ sont des atomes d'hydrogène, et R⁶ est le groupe méthyle, et R⁸ et R⁹ représentent ensemble un groupe -CH=CH-CH=CH-, ou bien
R³ et R⁵ sont des atomes d'hydrogène, et R⁶ et R⁷ forment ensemble un groupe -CH=CH-CH=CH-, et R⁸ et R⁹ forment ensemble un groupe -CH=CH-CH=CH-, ou bien
R³ et R⁴ sont des groupes tert-butyle, et R⁷ et R⁸ sont des atomes d'hydrogène, et R⁶ et R⁹ sont des groupes phényle, ou bien R⁶ est le groupe méthyle, et R⁹ est le groupe isopropyle ou orthotolyle, ou bien
R³ et R⁵ sont des groupes tert-butyle, et R⁸ est un atome d'hydrogène, et R⁶, R⁷ et R⁹ sont des groupes phényle, ou bien
R³ et R⁵ sont des groupes tert-butyle, et R⁷ est un atome d'hydrogène, et R⁶ est un groupe méthyle, et R⁸ et R⁹ forment ensemble un groupe -CH=CH-CH=CH-, ou bien
R³ et R⁵ sont des groupes tert-butyle, et R⁶ et R⁷ forment ensemble un groupe -CH=CH-CH=CH-, et R⁸ et R⁹ forment ensemble un groupe -CH=CH-CH=CH-.

6. Ligands de formule générale 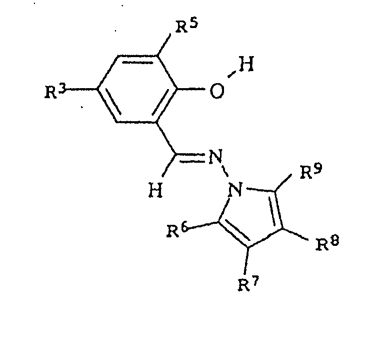 dans laquelle les variables sont définies comme suit :
R³ et R⁵ sont des atomes d'hydrogène, et R⁷ et R⁸ sont des atomes d'hydrogène, et R⁶ et R⁹ sont identiques et représentent des groupes méthyle ou phényle, ou bien R⁶ est le groupe méthyle et R⁹ est le groupe isopropyle, phényle ou orthotolyle, ou bien
R³, R⁵ et R⁸ sont des atomes d'hydrogène, et R⁶, R⁷ et R⁹ sont des groupes phényle, ou bien
R³, R⁵ et R⁷ sont des atomes d'hydrogène, et R⁶ est le groupe méthyle, et R⁸ et R⁹ représentent ensemble un groupe -CH=CH-CH=CH-, ou bien
R³ et R⁵ sont des atomes d'hydrogène, et R⁶ et R⁷ forment ensemble un groupe -CH=CH-CH=CH-, et R⁸ et R⁹ forment ensemble un groupe -CH=CH-CH=CH-, ou bien
R³ et R⁴ sont des groupes tert-butyle, et R⁷ et R⁸ sont des atomes d'hydrogène, et R⁶ et R⁹ sont des groupes phényle, ou bien R⁶ est le groupe méthyle, et R⁹ est le groupe isopropyle ou orthotolyle, ou bien
R³ et R⁵ sont des groupes tert-butyle, et R⁸ est un atome d'hydrogène, et R⁶, R⁷ et R⁹ sont des groupes phényle, ou bien
R³ et R⁵ sont des groupes tert-butyle, et R⁷ est un atome d'hydrogène, et R⁶ est un groupe méthyle, et R⁸ et R⁹ forment ensemble un groupe -CH=CH-CH=CH-, ou bien
R³ et R⁵ sont des groupes tert-butyle, et R⁶ et R⁷ forment ensemble un groupe -CH=CH-CH=CH-, et R⁸ et R⁹ forment ensemble un groupe -CH=CH-CH=CH-.

7. Procédé de préparation de composés complexes de formule générale I 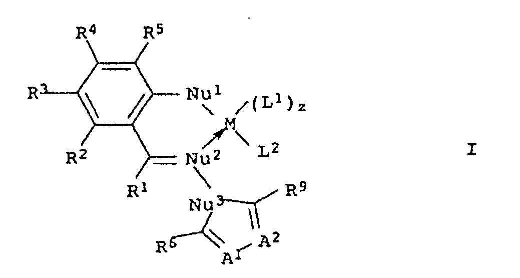 dans lesquels les variables sont définies comme suit :
M est un élément du 6ème au 10ème Groupe du Tableau Périodique des Eléments, à l'exception du nickel,
Nu¹ est choisi parmi O, S ou Se ;
Nu², Nu³ sont choisis parmi N ou P,
A¹ est N ou C-R⁷ ou Si-R⁷,
A² est N ou C-R⁸ ou Si-R⁸,
R¹ à R⁹ sont choisis parmi l'atome d'hydrogène ; les groupes alkyle en C₁-C₈, substitués ou non substitués ; les groupes alcényle en C₂-C₈, substitués ou non substitués, ayant 1 à 4 doubles liaisons isolées ou conjuguées ; les groupes cycloalkyle en C₃-C₁₂, substitués ou non substitués ; les groupes aralkyle en C₇-C₁₃ ; les groupes aryle en C₆-C₁₄, non substitués, ou une ou plusieurs fois substitués, par des substituants identiques ou différents choisis parmi les groupes alkyle en C₁-C₈ substitués ou non substitués, les groupes cycloalkyle en C₃-C₁₂, les groupes aralkyle en C₇-C₁₃, les groupes aryle en C₆-C₁₄, les atomes d'halogène, les groupes alcoxy en C₁-C₆ substitués ou non substitués, les groupes aryloxy en C₆-C₁₄, SiR¹⁰R¹¹R¹² ou O-SiR¹⁰R¹¹R¹² ;
les radicaux hétéroaryle azotés à cinq à six chaînons, non substitués, ou une ou plusieurs fois substitués par des substituants identiques ou différents choisis parmi les groupes alkyle en C₁-C₈ substitués ou non substitués, les groupes cycloalkyle en C₃-C₁₂, les groupes aralkyle en C₇-C₁₃, les groupes aryle en C₆-C₁₄, les atomes d'halogène, les groupes alcoxy en C₁-C₆, les groupes aryloxy en C₆-C₁₄, SiR¹⁰R¹¹R¹² ou O-SiR¹⁰R¹¹R¹² ;
où les radicaux spatialement voisins R¹ à R⁹ peuvent être reliés les uns aux autres pour donner un noyau à 5 à 12 chaînons ;
L¹ est un ligand neutre organique ou inorganique,
L² est un ligand anionique organique ou inorganique, L¹ et L² pouvant être reliés l'un à l'autre par une ou plusieurs liaisons covalentes,
z est un nombre entier de 1 à 3,
R¹⁰ à R¹² sont identiques ou différents et sont choisis parmi l'atome d'hydrogène, les groupes alkyle en C₁-C₈, cycloalkyle en C₃-C₁₂, aralkyle en C₇-C₁₃ ou aryle en C₆-C₁₄,
**caractérisé en ce qu'**on déprotone d'abord, à l'aide d'une base, un ligand de formule générale II 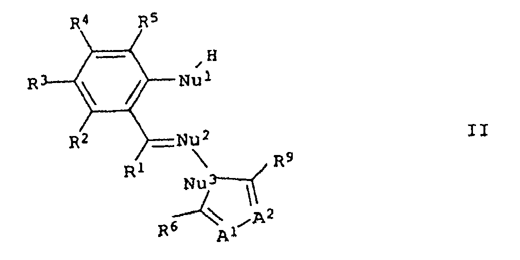
puis on le fait réagir avec 0,2 à 5 équivalents d'un composé métallique MX₄, MX₃, ML¹L² ou MX₂, où X est un atome d'halogène, un groupe alkyle en C₁-C₈, cycloalkyle en C₃-C₁₂, aralkyle en C₇-C₁₃ ou aryle en C₆-C₁₄, et MX₂, MX₃ ou MX₄ est stabilisé par d'autres ligands neutres.

8. Procédé de préparation de composés complexes de formule générale I selon la revendication 7, **caractérisé en ce que** M est un élément du 6ème au 10ème Groupe du Tableau Périodique des Eléments, à l'exception du nickel, et que MX₂, MX₃ ou MX₄ est en option stabilisé par d'autres ligands neutres.

9. Procédé de préparation de composés complexes de formule générale I selon la revendication 7, **caractérisé en ce qu'**on utilise en tant que base le méthyllithiyum, l'éthyllithium, le n-butyllithium, le sec-butyllithium, le tert-butyllithium ou l'hexyllithium, des composés de Grignard, l'amidure de lithium, l'amidure de potassium, l'hydrure de potassium ou le diisopropylamidure de lithium, et MX₂, MX₃ ou MX₄ est en option stabilisé par d'autres ligands neutres.

10. Procédé de polymérisation ou de copolymérisation d'oléfines par utilisation de composés complexes selon les revendications 1 à 4.

11. Procédé de préparation d'un catalyseur supporté pour la polymérisation ou la copolymérisation d'oléfines, **caractérisé en ce qu'**on dépose sur un support solide un ou plusieurs composés complexes selon les revendications 1 à 4, et en option un activateur.

12. Catalyseur supporté pour la polymérisation ou la copolymérisation d'oléfines selon la revendication 11.

13. Procédé de polymérisation ou de copolymérisation d'oléfines par utilisation d'un catalyseur supporté selon la revendication 11.

14. Procédé de polymérisation en émulsion ou de copolymérisation en émulsion de l'éthylène ou d'autres 1-oléfines et en option d'autres oléfines, par utilisation d'un composé complexe de formule générale I, dans laquelle M est un élément du 6ème au 10ème Groupe du Tableau Périodique des Eléments.
